(19) Europäisches Patentamt / European Patent Office / Office européen des brevets

(11) **EP 3 978 071 A1**

(12) **EUROPEAN PATENT APPLICATION**

(43) Date of publication:
**06.04.2022 Bulletin 2022/14**

(21) Application number: **20199710.3**

(22) Date of filing: **01.10.2020**

(51) International Patent Classification (IPC):
***A61N 5/06*** *(2006.01)*

(52) Cooperative Patent Classification (CPC):
**A61N 5/0616; A61N 5/0613;** A61N 2005/0626

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **G Life**
**01710 Thoiry (FR)**

(72) Inventors:
• **GERELLI, Emmanuel**
**1046 Rueyres (CH)**
• **JONIOVA, Jaroslava**
**1007 Lausanne (CH)**
• **GERELLI, Sebastien**
**01710 Thoiry (FR)**
• **WAGNIÈRES, Georges**
**1095 Lutry (CH)**

(74) Representative: **Grosfillier, Philippe**
**ANDRE ROLAND S.A.**
**IP Attorneys & Services**
**Avenue Collonges 21**
**1004 Lausanne (CH)**

(54) **DEVICE FOR APPLYING PHOTOBIOMODULATION**

(57) A device for applying Photobiomodulation (PBM) on a biological object comprising an EM source delivering light with an adequate temporal evolution of its optical power, said device also comprising a processing and/or a light control unit that determines the adequate temporal evolution of the optical power on the basis of the biological object optical coefficients and the light delivery geometry on/in the biological object, characterized by the fact that the PBM effects are induced by the generation of a specific fluence rate during a specific time, successively in each parts of the volume of the biological object.

The invention also relates to different methods for applying Photobiomodulation (PBM) on a biological object comprising an EM source delivering light with an adequate temporal evolution of its optical power.

(Model and Monte-Carlo simulation: $\mu_a$ =0.27 mm$^{-1}$, $\mu_s$ =18.7mm$^{-1}$, g=0.81, k=4.87, $\mu_{eff}^{-1}$ =0.57 mm) (S.L. JACQUES)

$$\frac{F(z)}{E} = e^{-\mu_{eff} z}$$

**Fig. 1**

**EP 3 978 071 A1**

**Description**

**Field of invention**

The present invention generally relates to photobiomodulation and more precisely to devices and methods for applying photobiomodulation.

**State of the art**

Definitions

The following definitions apply to the present document.

- Light: Electromagnetic radiations with wavelengths ranging between 250 nm and 3 $\mu$m.
- Irradiance or primary incidence **E**[W/m$^2$]: The irradiance describes the power per unit surface directly received from a source.
- Radiance **L**[W/(m$^2$.sr)]: The radiance is the power of light that passes through or is emitted from a unit surface area and propagates within a unit solid angle in a specified direction.
- Fluence rate **F**[W/m$^2$]: The fluence rate is the power entering a sphere presenting a unit cross-section. It takes into account diffusion and/or scattering effects in the target environment. The fluence rate is measured with an isotropic power meter. It takes into account the direct flux (the irradiance) as well as the scattering and diffusion contributions. Like the Fluence (see below) this term is of fundamental importance in dosimetry where multiple scattering and diffusion in the target tissue are of great importance.
- Fluence or light dose $\Psi$[J/m$^2$]: Is the time integral of the fluence rate. Therefore, the fluence is the energy entering a sphere presenting a unit cross-section.
- Absorption coefficient $\mu_a$[m$^{-1}$]: Inverse of the mean free path before photon absorption
- Scattering coefficient $\mu_s$[m$^{-1}$]: Inverse of the mean free path between photon scattering
- Reduced scattering coefficient $\mu_s$'[m$^{-1}$]: $\mu_s$'= $\mu s$(1-g)
- Anisotropy factor **g**[--]: g is equal to the mean value of cos$\theta$, where $\theta$ is the deflection angle of a photon scattered by a particle.
- Effective attenuation coefficient $\mu_{eff}$[m$^{-1}$]: $\mu_{eff}$= $(3\mu_a(\mu_a+\mu_s'))^{1/2}$

Photobiomodulation, named PBM in the present document, refers to the treatment of biological objects, such as a tissue, with certain wavelength(s) of light to facilitate regeneration and remodeling, resolve inflammation, reduce edema, relieve pain, modulate the immune system and the metabolism. Clinical applications include soft tissue injuries, chronic pain, wound healing, nerve regeneration, blood treatment and possibly even resolving viral and bacterial infections. Many conditions are associated with perturbations of the metabolism, including deficiencies of the mitochondrial respiration. These conditions include neurodegenerative diseases (Parkinson's, Alzheimer's and Huntington's diseases), atherosclerosis, certain forms of diabetes, autoimmune diseases, cancer, chronic wounds, damages resulting from ischemia-reperfusions and chronic or acute inflammation like the acute respiratory distress syndrome (ARDS). It is also well known that the metabolism is significantly altered in the cases of stroke, heart attack, grafts or ischemic wounds, among other. As an example, the inventors have shown that mitochondrial respiration play an important role in the heart remodeling [Kindo, 2016], particularly the cardiac metabolism reacted to a parietal stress by a mitochondrial dysfunction [Kindo, 2012].

Therefore, strategies to normalize, restore and/or increase the metabolism are of high interest to treat and characterize numerous conditions. PBM therapy is one of these strategies [Hamblin 2017; Hamblin 2018].

PBM therapy is based on the administration of light at low (sub-thermal) irradiance, mostly at wavelengths ranging between 600 and 900 nm, a spectral window corresponding to the maximal light penetration depth in most soft tissues. PBM has a broad range of molecular, cellular, and tissular effects [Hamblin 2017; Hamblin 2018].

However, its mechanisms are not yet fully understood. Moreover, PBM conditions are very rarely optimized and/or mastered. Based on the studies conducted by several groups [Hamblin 2017; Hamblin 2018] and, most importantly, in vitro and in vivo observations carried out by the inventors, one can conclude that PBM generates several positive effects, in particular:

    a) An increase of the tissue oxygen ($O_2$) consumption following or during hypoxia,
    b) A stimulation of angiogenesis,
    c) A stimulation of regeneration processes at the cellular level,
    d) An increase, following an application of 5-aminolevulinic acid (ALA), of the endogenous production of protoporphyrin IX (PpIX) [Sachar,2016], which can be used as an $O_2$ sensor. Several formulations of ALA to induced PpIX as photosensitizer and fluorescing markers are approved for cancer therapy and detection, respectively.

e) An increase of the ATP production, indicating an improved metabolic activity,

f) A modulation of reactive oxygen species (ROS)

g) A modulation of reactive nitrogen species (RNS)

h) A rescuing of cells subject to an intoxication.

These observations probably result from a stimulation of the mitochondrial metabolic activities and are of high interest for numerous medical applications, including those mentioned above [Hamblin 2017; Hamblin 2018].

PBM is, in particular, of interest for the treatment of myocardial infarction (MI) [Liebert, 2017], which is one the most common acute pathologies. It represents a major cause of death worldwide. At present, the treatments of choice for patients suffering from MI to limit its size and reduce acute myocardial ischemic injury are time consuming, have side effects and limited efficacies. They consist of either primary percutaneous coronary intervention or thrombolytic therapy. Moreover, the treatment itself (process of reperfusion) can be the cause of death of cardiomyocytes until days after the treatment, a process also known as myocardial reperfusion injury, for which, up to this date, there is still no effective treatment [Chouchani, 2016; Ferrari, 2017; Kalogeris, 2017].

The inventors have shown (see below) that the control of the light dosimetry (fluence rate [mW/cm$^2$]; light dose [J/cm$^2$]) and spectroscopy (wavelength(s)) is crucial to induce optimal PBM effects. This observation is very important since the PBM effects are known to be <u>bimodal</u>, i.e. too high or too low fluence rates and/or light doses significantly reduce the PBM effects and are therefore frequently associated to the Arndt-Schultz rule observed in pharmacology. This bimodal response has been reported by numerous groups looking at various "standard" effects (mitochondria membrane potential; ATP production; etc) [Huang 2009; Hamblin 2017; Hamblin 2018]. The inventors confirmed and better characterized this bimodal response looking at the PBM effects on the endogenous production of PpIX in different cell lines, including glioma cells and human cardiomyocytes (HCM).

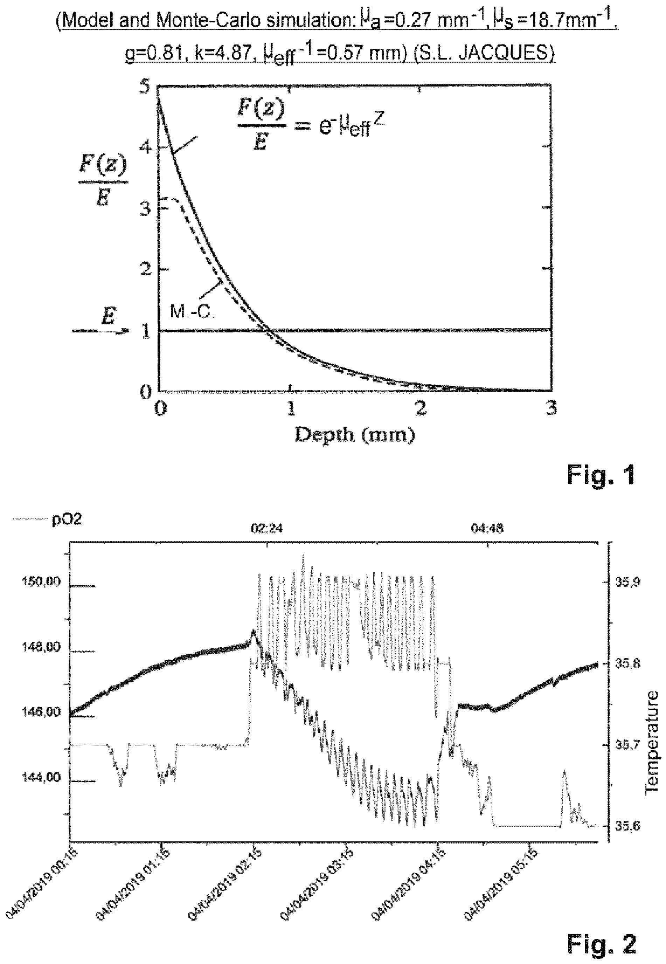

It is also established that the optical properties of biological tissues, described mostly by their absorption and scattering coefficients, have an important impact on the propagation of the light around a light source [Tuchin, 2015; Hamblin 2017; Hamblin 2018]. In general, the fluence rate (and the light dose) decreases with the distance from the light source due to the absorption and scattering of the light in the tissue (see Figure 1). Therefore, the fluence rate and/or the light dose in the tissues are, in most situations, never optimal at the same time in different locations in the tissues treated by PBM.

The strong bimodal dependence of the PBM effects on the radiometric conditions combined with: i) the heterogeneous distribution of the light in the tissues treated by PBM, and ii) the very limited control of the light delivery and dosimetry by the vast majority of the research or clinical groups active in this field explain the limited and contradictory outcomes reported in the literature [Chung 2012]. This situation also explains why PBM therapy is poorly used at present.

### List of figures

**Figure 1:** Evolution of the fluence rate/irradiance ratio versus the depth in a semi-infinite tissue for a "broad", collimated and perpendicular illumination of the air-tissue interface. The continuous curve is the solution of the diffusion approximation, whereas the dashed curve is the solution of a Monte-Carlo computer-based simulation, where $\mu_a$ and $\mu_s$ are the absorption and scattering coefficients, respectively. $\mu_{eff}$ is the effective attenuation coefficient, g is the anisotropy factor, whereas k is the pre-exponential factor resulting from the backscattering of light. Derived from [Jacques, 2010].

**Figure 2:** Temporal evolution of the $pO_2$ (black curve; mmHg) and temperature (grey curve; °C) measured above a monolayer of HCM cells subject to metabolic oscillations.

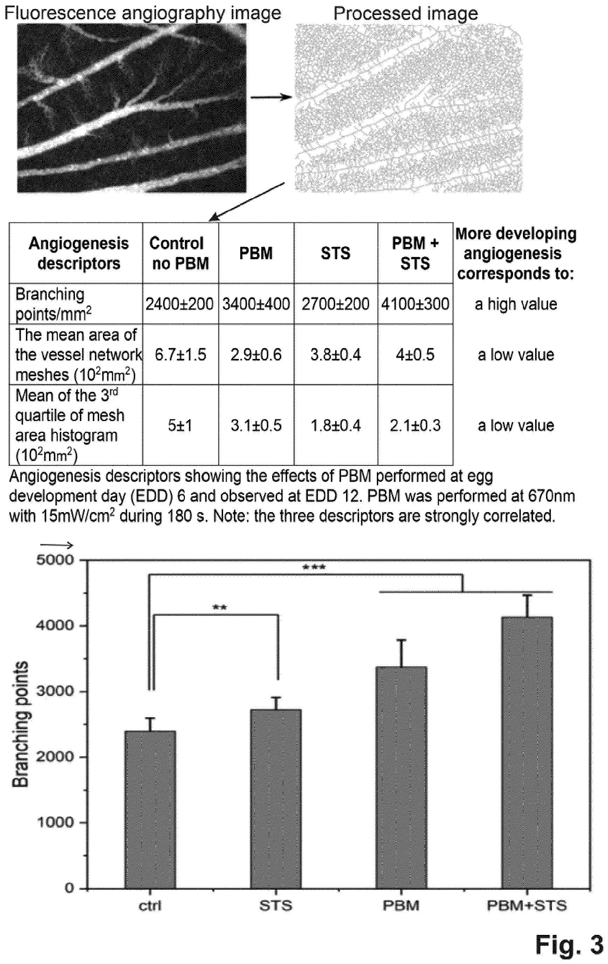

**Figure 3:** Synergic effect of STS within PBM on angiogenesis.

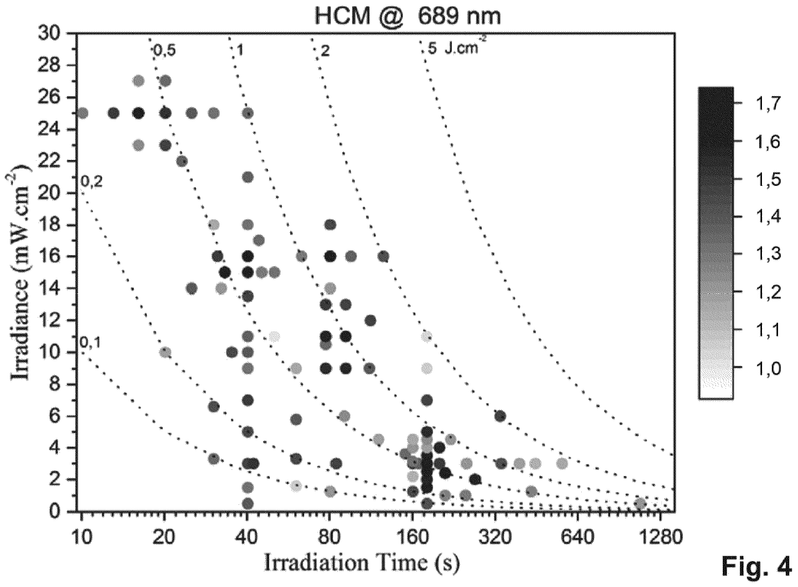

**Figure 4:** Relative increase of the PpIX fluorescence, which reflects the metabolic activity, observed in human cardiomyocytes (HCM) relative to control (no PBM) samples. The values of the ratio "treated by PBM / controls" are given by the monochrome bar.

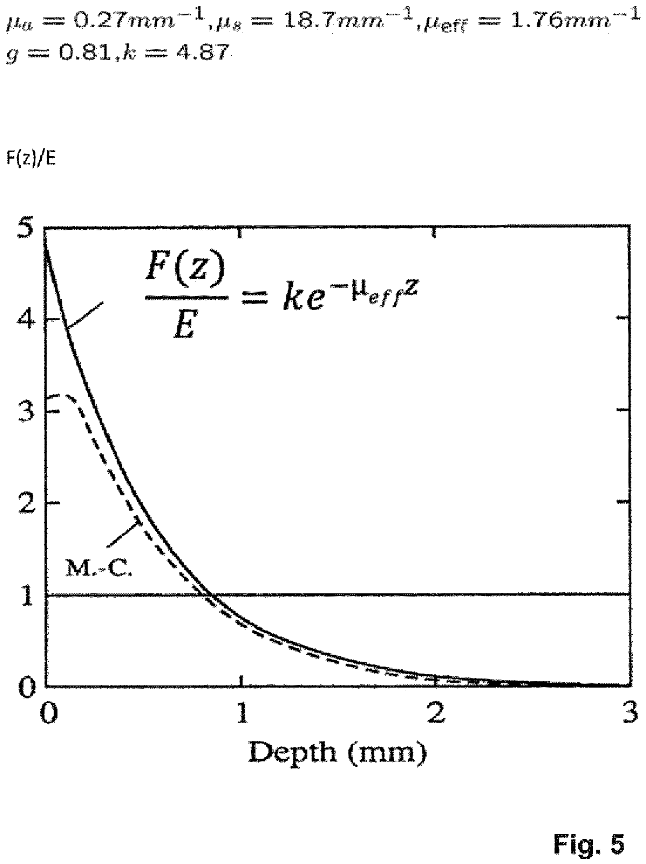

**Figure 5:** Evolution of the fluence rate/irradiance ratio (F/E) versus the depth in a semi-infinite tissue for a "broad", collimated and perpendicular illumination of the air-tissue interface. The continuous curve is the solution of the diffusion approximation, whereas the dashed curve is the solution of a Monte-Carlo computer-based simulation, where $\mu_a$ and $\mu_s$ are the absorption and scattering coefficients, respectively. $\mu_{eff}$ is the effective attenuation coefficient, g is the anisotropy factor, whereas k, which depends on the refractive index matching conditions (ntissue/nair = 1.37) as well as the optical coefficients, is the pre-exponential factor resulting from the backscattering of light. Derived from [Jacques, 2010].

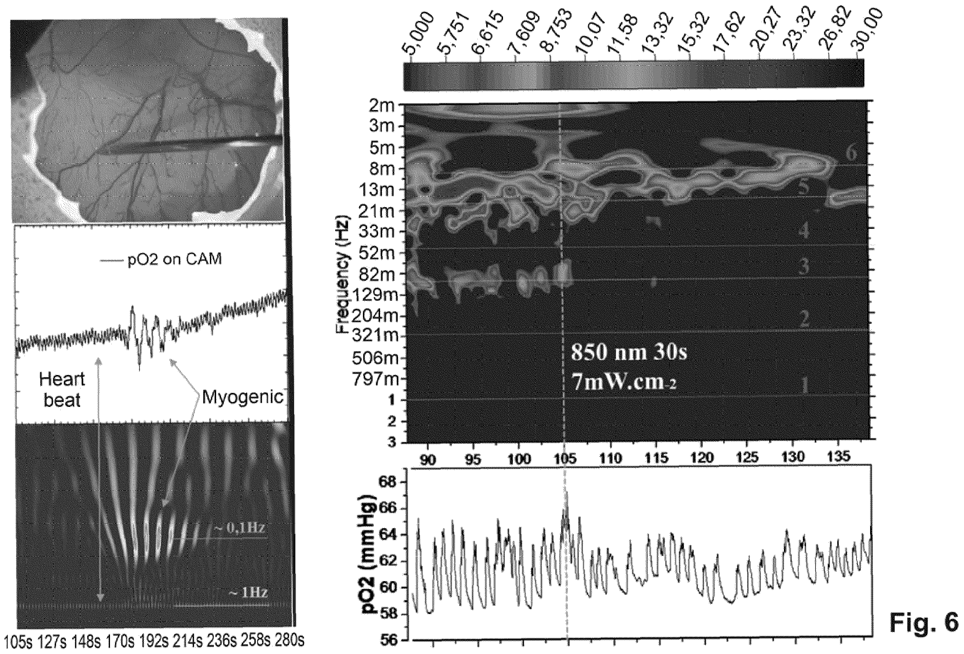

**Figure 6:** Frequency analysis of the $pO_2$ in the CAM at EDD 7. Left up: Image of the experiment showing the metallic Clark's probe applied against a blood vessel. Left middle: $pO_2$ signal (acquired at 50 Hz) and (Left down) the associated spectrum based on wavelet analysis (the vertical scale is the frequency, and the monochrome level represents the oscillation amplitude, the horizontal scale is the time in seconds). Right bottom: $pO_2$ signal (acquired at 1 Hz) and (right up;

the monochrome level represents represents the oscillation amplitude) associated spectrum resulting from the wavelets analysis. The horizontal axis is the time given in minutes. A strong activation of the $pO_2$ tone is observed 25 minutes (time out of the scale) after a topical application of NaHS (10 $\mu$l - 1$\mu$M), which is deactivated by PBM (850 nm, 7 mW.cm$^2$, 30 s) at time 105 min (see myogenic signal). The horizontal lines reported on the spectrum indicate specific frequencies. 1 - Cardiac, 2 - Respiratory, 3 - Myogenic, 4 - Neurogenic, 5 - eNOS dept, 6 - eNOS indept (probably prostaglandin [Shiogai and al.]). Cardiac frequency cannot be resolve within the sampling. Respiratory and neurogenic bands do not exist at this EDD.

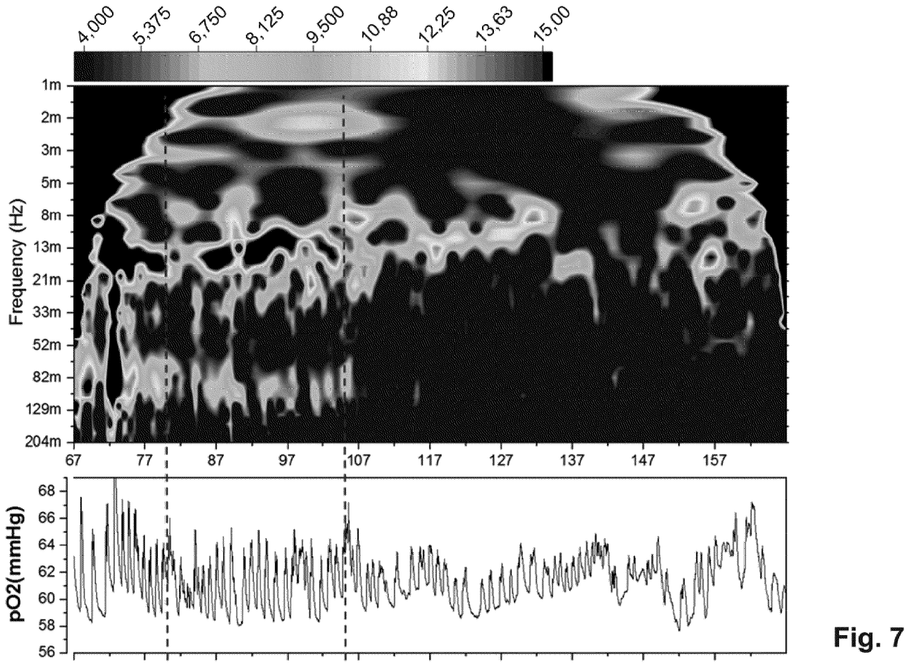

**Figure 7:** Enlargement of the experiment presented in figure 6. The horizontal axis gives the time in minutes.

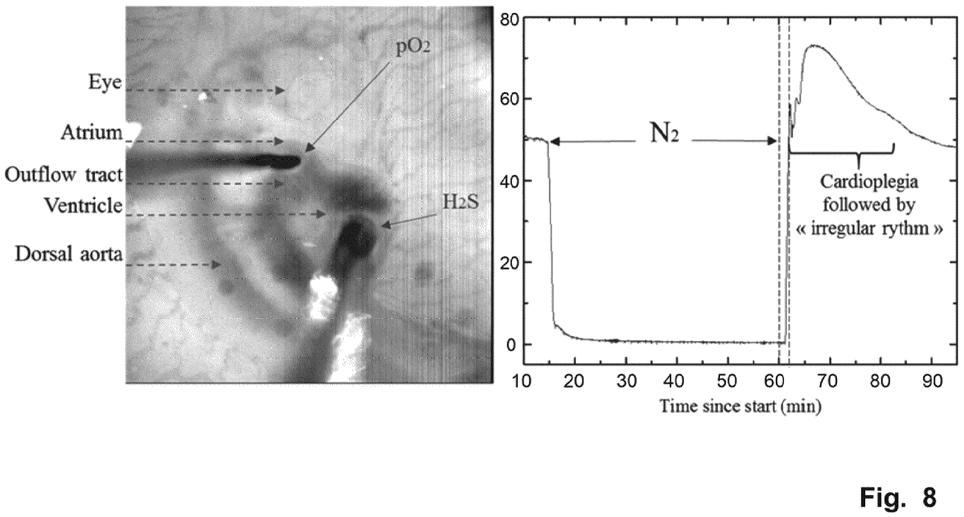

**Figure 8:** Left: Image of the in ovo anoxia reoxygenation experiment. Right: $pO_2$ signal (OX-100 $\mu$m Unisense®) recorded all along the experiment (Vertical axis: mmHg). The hypoxia due to the flushing of $N_2$ (up to 60 minutes) is followed by a reoxygenation.

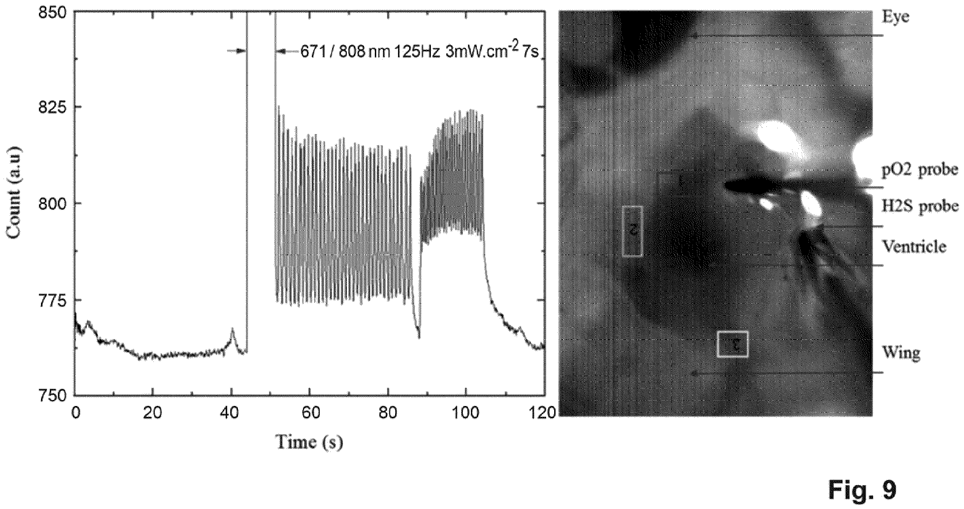

**Figure 9:** Stimulation by PBM of a chicken embryo's heart at EDD 5 during an anoxic cardioplegia. Left: Time course measurement of the heartbeat (assessed by the change of the heart reflectivity in the region of interest defined by the green rectangle presented on the image showing the embryo (right). The heartbeat is stopped between 0 and 45 s. Then, a PBM illumination during 7 seconds re-activated the heart beat during more than 1 min.

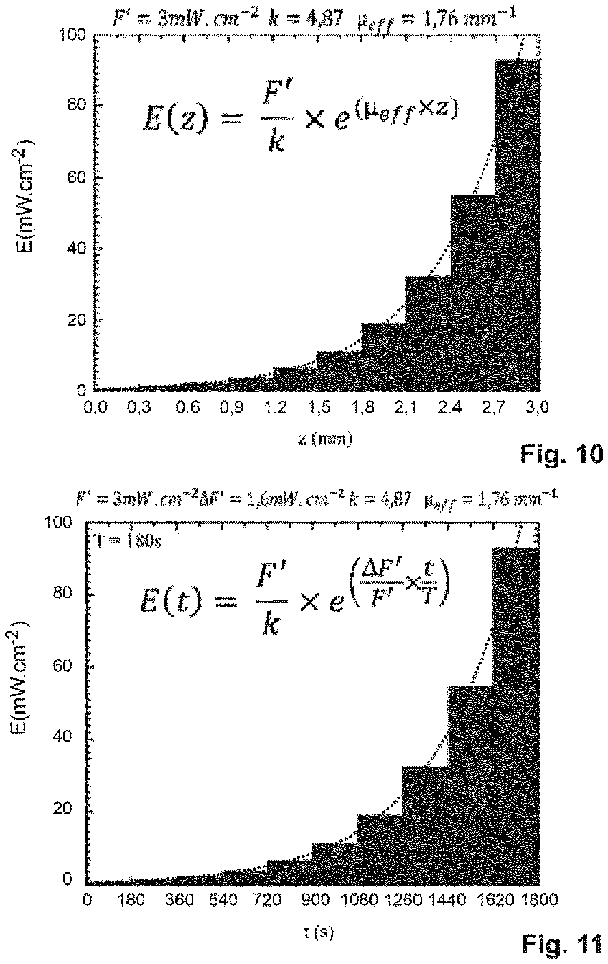

**Figure 10:** Spatial evolution of the irradiance E in a semi-infinite tissue for a "broad", collimated and perpendicular illumination of the air-tissue interface. $\Delta z$=0,3 mm; n=10; ntissue/nair = 1.37; T=180 s; $\Delta F$'= 1.6 mW/cm$^2$; The dotted curve corresponds to the continuous fit of the "step-based" evolution of the irradiance. The analytical expression of this fit is presented as insert in this figure. This illustrates that E may be changed continuously instead of incrementally.

**Figure 11:** Temporal evolution of the irradiance E in a semi-infinite tissue for a "broad", collimated and perpendicular illumination of the air-tissue interface. $\Delta z$=0,3 mm; n=10; $n_{tissue}/n_{air}$ = 1.37; T=180 s; $\Delta F$'= 1.6 mW/cm$^2$; The dotted curve corresponds to the continuous fit of the "step-based" evolution of the irradiance. The analytical expression of this fit is presented as insert in this figure. This illustrates that E may be changed continuously instead of incrementally.

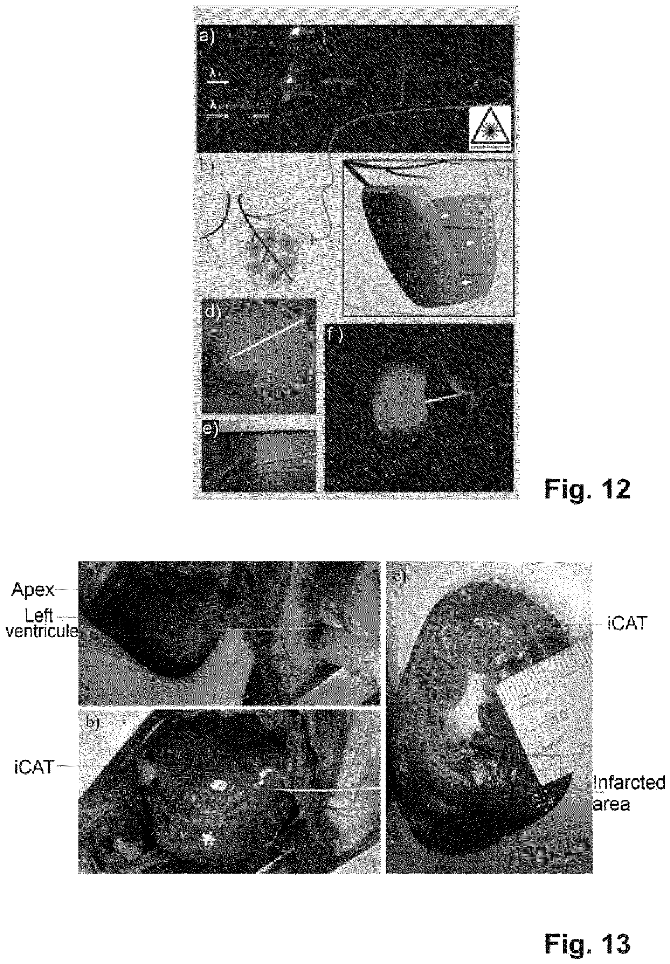

**Figure 12:** Overview of a trans-myocardial implantation at 90° during coronary thrombosis (b). of cylindrical distributors (d) connected to an optical source (a) are placed trans-myocardially (c) with a predefined pattern and spacing through a mask. In (e) the light distributor, the iCAT® catheter and the guide to be inserted into the catheter for transfixion. In (f) image illustrating the propagation of light from an isotropic distributor (sphere of 0.8mm diameter) implanted in the center of an ex vivo swine left ventricular myocardium (652 nm - 100mW).

**Figure 13:** Implantation of an iCAT® catheter all along the damage left ventricle of the swine heart in situ after a sternotomy. A) Catheter was introducing from the apex side until it come out of the top of left ventricle B). C) Heart cross section after the heart excision without removing the catheter. The catheter, here is well placed close to the middle of the myocardium thickness.

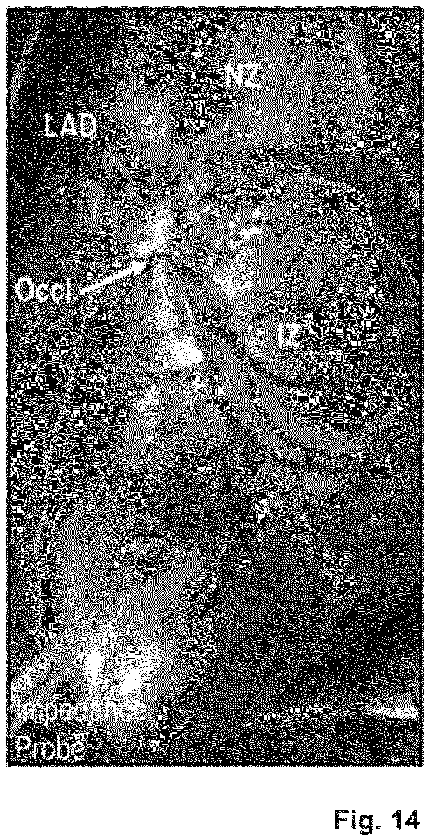

**Figure 14:** Visual localization of the ischemic area (IZ) after the occlusion of the descending left anterior coronary artery (LAD) which partially perfuses the left ventricle during an opened chest surgery on pig. The IZ is easily differentiated from non-ischemic area (NZ). An electrical impedance sensor can also be used to characterize the ischemic area.

AJP- Heart Circ Physiol

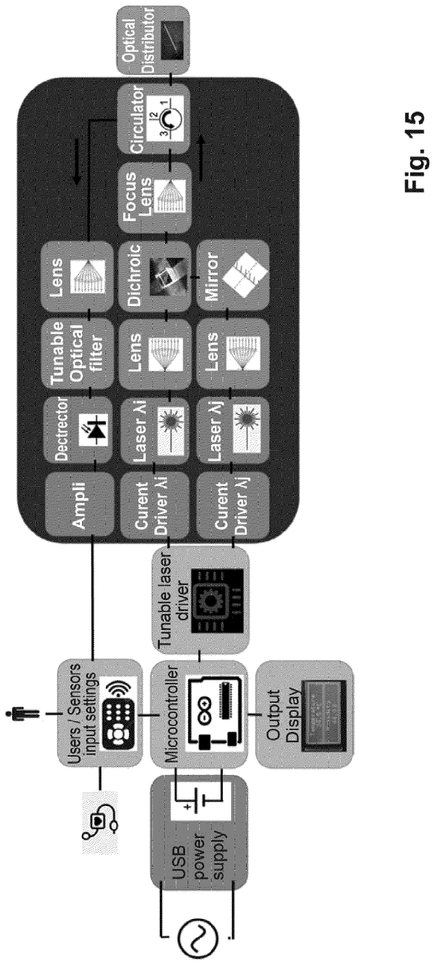

**Figure 15:** Simplified diagram illustrating an example of a part of the device supplying light to an optical distributor.

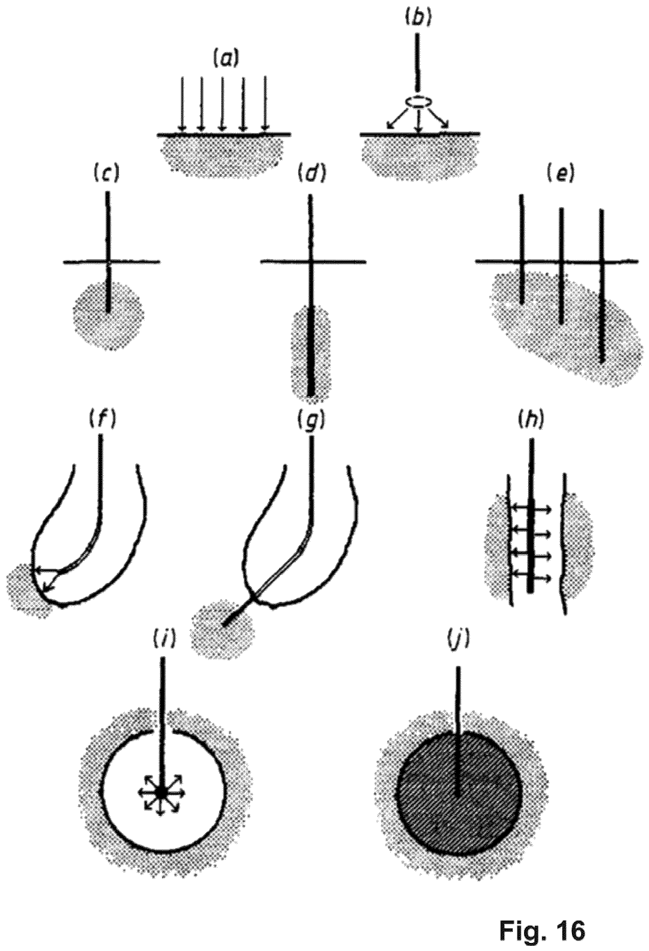

**Figure 16:** Examples of the irradiation geometries used in photobiomodulation or LLLT. The stippled areas represent schematically the pattern of fluence rate in tissue. (a), (b) Surface irradiation from broad beam or lens-tipped fiber. (c)-(e) Interstitial irradiation with cut-end or cylindrical fibers, (f)-(h) Intracavitary and intralumenal irradiation. (i), (j) Intracavitary whole-surface irradiation using an isotropically-tipped fiber or a light-diffusing liquid (shaded). Source: Wilson, 1986.

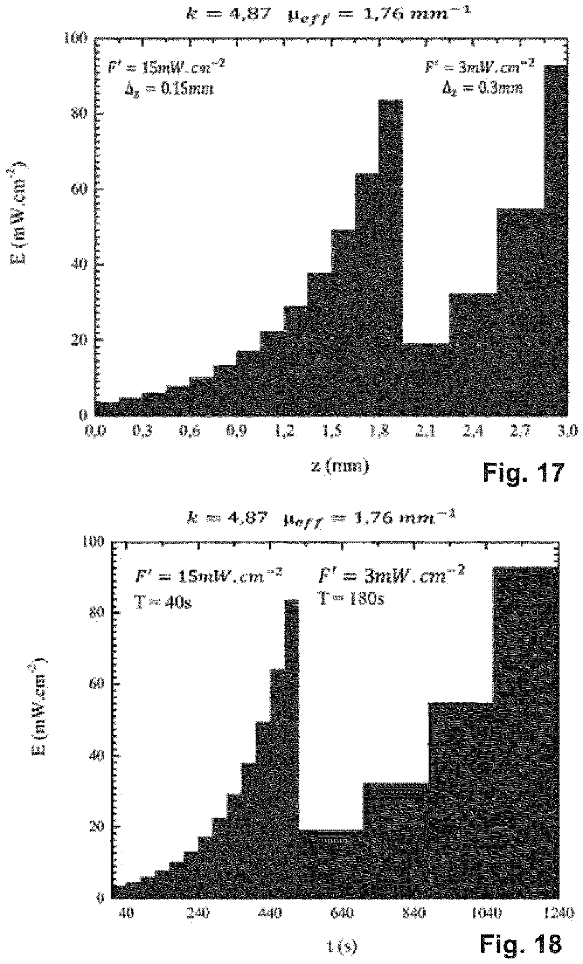

**Figure 17:** Spatial evolution of the irradiance E in a semi-infinite tissue for a "broad", collimated and perpendicular illumination of the air-tissue interface. The spatial evolution of E is optimized in such a way that its value never exceeds 100 mW/cm$^2$, while minimizing the total illumination time. The idea is to illuminate the sample exploiting two PBM hot spots visible in figure 4, i.e. generating fluence rates of 15 and 3 mW/cm2 during 40 and 180 s (values of T), respectively. The corresponding values for $\Delta F$' $\Delta z$ and n (the number of steps) are respectively:

- 4 mW/cm$^2$, 0,15 mm and 13 for T = 40 s
- 1.6 mW/cm$^2$, 0.3 mm and 4 for T = 180 s.
  $n_{tissue}/n_{air}$ = 1.37.

**Figure 18:** Temporal evolution of the irradiance E in a semi-infinite tissue for a "broad", collimated and perpendicular illumination of the air-tissue interface. The spatial evolution of E is optimized in such a way that its value never exceeds 100 mW/cm$^2$, while minimizing the total illumination time. The idea is to illuminate the sample exploiting two PBM hot spots visible in figure 4, i.e. generating fluence rates of 15 and 3 mW/cm2 during 40 and 180 s (values of T), respectively. The corresponding values for $\Delta$F' $\Delta$z and n (the number of steps) are respectively:

- 4 mW/cm$^2$, 0,15 mm and 13 for T = 40 s
- 1.6 mW/cm$^2$, 0.3 mm and 4 for T = 180 s.
  $n_{tissue}/n_{air}$ = 1.37.

The "step-based" evolution of the irradiance can be fitted by the analytical expression presented as insert in figure 11.

**General description of the invention**

An object of the present invention is to provide an improved PBM for biological objects such as tissues.

Another object of the present invention is to provide an efficient treatment of MI with PBM.

According to the invention, the device for applying PBM comprises an EM source delivering light with an adequate temporal evolution of its optical power. The device also comprises a processing and/or a light control unit that determines the adequate temporal evolution of the optical power on the basis of the biological object optical coefficients and the light delivery geometry on/in the biological object. The device is characterized by the fact that the PBM effects are induced by the generation of a specific fluence rate during a specific time successively in each parts of the volume of the biological object.

The invention also includes a method for applying PBM on a biological object wherein light is delivered with an adequate temporal evolution of the optical power, this power being determined on the basis of the biological object optical coefficients and the light delivery geometry on/in the biological object. The method is characterized by the fact that the PBM effects are induced by the generation of a specific fluence rate during a specific time successively in each parts of the volume of the biological object.

The invention furthermore relates to a method for applying PBM on a biological object wherein light is delivered with an adequate temporal evolution of the optical power , characterized by the fact that the optical power, the delivery of light and/or the irradiance is based on the measurement, or on a frequency analysis of fluctuations, of one or several parameters reflecting the metabolic activity of the biological object.

The invention also encompasses a method for applying PBM on a biological object wherein light is delivered with an adequate temporal evolution of the optical power , characterized by the fact that the optical power, the delivery of light and/or the irradiance is based on a frequency analysis of fluctuations of one or several parameters reflecting the PBM effects on the biological object.

Optionally, the light power emitted by this device, or the light applied during the application method of PBM mentioned above, is adapted on the basis of feedback observables (see the list given below) to optimize the PBM effects.

List of feedback observables:

a) The temperature.

Figure 2 presents the evolution of the temperature measured with a thermocouple and a probe used to measure the oxygen consumption rate (OCR), which reflects the metabolic activity, in an experimental setup developed by the inventors. This setup consists in a monolayer of Human CardioMiocytes (HCM) positioned at the bottom of a petri dish which was covered with a 15 mm thick layer of physiologic water. The thermocouple and the probe to measure the OCR as well as the partial pressure of oxygen ($pO_2$) were both positioned 1 mm above the cell monolayer.

As can be seen on figure 2, significant and easily measurable changes of the temperature are observed while the $pO_2$, and hence the OCR, are changing. The temperature increases with an increasing activity of the OCR. Interestingly, the high-frequency oscillations observed for the OCR and the temperature after a maximal value of the $pO_2$ are in phase and synchronous.

b) The tissue autofluorescence reflecting the redox state of enzymes involved in the metabolism.

Oxidation is the main process producing the necessary energy in cells. It can occur in aerobic or anaerobic conditions. In many situations, biological oxidation starts with substrate dehydrogenation, i.e. the displacement of two hydrogen atoms, whereas coenzymes such as NAD+, NADP+ and FAD serve as acceptors of these atoms. Since the cellular concentration of these coenzymes is low, they must be recycled by re-oxidization. Therefore, these coenzymes serve as primary donor and acceptor in the process of oxidative phosphorylation (OXPHOS) [Ferraresi, 2012]. Since NADH and FAD are bound to many enzymes involved in metabolic pathways [Alberts, 2002], the relative ratio between the NADH and FAD binding sites changes as well when the cells are switching their metabolism [Banerjee, 1989]. Hence, cell responses to changes of the $O_2$ level (change of metabolic activity) resulting from PBM can be monitored looking at their effects on FAD and NADH.

These coenzymes can be studied non-destructively looking at their autofluorescence, i.e. without the addition of exogenous probes [Ramanujam, 2001]. One of the most common optical techniques giving information about the metabolic state of cells is based on the determination of the redox ratio of FAD and NADH by fluorescence spectroscopy [Chance, 1979; Walsh, 2012; Blacker, 2016], in particular time-resolved fluorescence spectroscopy [Skala, 2007; Skala, 2010; Kalinina, 2016; Walsh, 2013], a field corresponding to the expertise of the inventors since more than two decades [Wagnières, 1998]. For instance, in cancer cells, an increase of cellular metabolism is usually indicated by a decrease of the redox ratio [Chance, 1989].

Therefore, steady-state and/or time-resolved fluorescence spectroscopy (or imaging) of the tissue autofluorescence is an interesting feedback observable to monitor or adapt the light dose used for PBM. Interestingly, the combined use of this approach with direct $O_2$ sensing based on the time-resolved luminescence spectroscopy of molecular probes (PPIX or exogenous pO2 probes as proposed by Kalinina et al. [Kalinina, 2016]) or interstitial Clark's probes, provide unique information on the PBM effects. Monitoring these parameters is minimally invasive and fast.

c) The assessment of the hemoglobin saturation.

In normal conditions, the body maintains a stable level of oxygen saturation for the most part by chemical processes of aerobic metabolism associated with breathing. However, it is well known that the hemoglobin saturation can change for different metabolic activities.

Since many methods are well established to measure the hemoglobin saturation, applying this approach to monitor the changes of metabolic activities induced by PBM is of high interest.

In addition, since several gazes can be endogenously produced and diffused within the tissue and the circulating blood, and can bind to various metalloproteins, which present strong optical absorption bands, for instance, NO or $H_2S$ can bind deoxyhemoglobin to create nitrosyl hemoglobin or sulfhemoglobin which decrease the level of available deoxyhemoglobin, and since PBM can induce photodissociation of metalloproteins as hemoglobin, especially nitrosyl [Lohr, 2009], and since the changes of these different forms of "hemoglobin" can be measured (via optical absorption measurement [Van leeuwen, 2017]), monitoring the changes of the metabolic activities induced by PBM through the assessment of these various metalloproteins complexes is of high interest.

d) The pH.

It is well known that glycolysis, which is the metabolic pathway that converts glucose into pyruvate, leads to an acidification of the extracellular surrounding media. This acidification frequently results from the excretion of lactic acid after its conversion from pyruvate [Wu, 2007]. Since many methods are well established to measure the pH in tissues, applying this approach to monitor the changes of metabolic activities induced by PBM is of high interest.

As tissular (or saliva) lactate concentration can be constantly assess with minimally invasive device such subcutaneous microneedle [Tsurukoa, 2016] or in mouth, this approach can be used to monitor the changes of metabolic activities induced by PBM.

e) The concentration of ROS.

Reactive oxygen species (ROS) are chemically reactive chemical species containing oxygen. Examples include peroxides, superoxide, hydroxyl radical, singlet oxygen, [Hayyan, 2016] and alpha-oxygen. In a biological context, ROS are formed as a natural byproduct of the normal metabolism of oxygen and have important roles in cell signaling and homeostasis [Devasagayam, 2004]. ROS are produced during a variety of biochemical reactions within the cell and within organelles such as mitochondria, peroxisomes, and endoplasmic reticulum.

Effects of ROS on cell metabolism are well documented in a variety of species [Nachiappan, 2010]. These include not only roles in apoptosis (programmed cell death) but also positive effects such as the induction of host defense genes and mobilization of ion transport systems. This implicates them in control of cellular function. In particular, platelets involved in wound repair and blood homeostasis release ROS to recruit additional platelets to sites of injury. These also provide a link to the adaptive immune system via the recruitment of leukocytes.

Abnormal levels of ROS are implicated in numerous pathologies through a strong modulation of various biological cascades [Sies, 2020]. Interestingly ROS level are also primordial for cell reprograming [Bigarella, 2014; Zhou, 2016] and tissular remodeling. For instance, their production kinetics depend on a broad spectrum of extrinsic or intrinsic repetitive stimulus such as hormones secretion or mechanical forces (like vascular shear stress), which influence directly the tissular behavior and properties [Hwang, 2003; Brandes, 2014], and finally phenotypic aspects.

Since many methods are well established to measure the ROS in tissues, applying this approach to monitor the changes of metabolic activities induced by PBM is of high interest.

As ROS and reactive nitrogen species (RNS) are intricate by nature [Moldogazieva, 2018] and since many methods to assess to RNS are well established [Griendling, 2016], the termed of reactive oxygen and nitrogen species RONS should be used here. Actually, in a full extends, the term should be reactive oxygen and nitrogen and sulfur species (RONSS).

f) The level of H$_2$S.

Hydrogen sulfide (H$_2$S) exert a wide range of actions on the whole organism. It is an epigenetic modulator inducing histone modification particularly via DNA demethylation, a process which permit cell differentiation [Yang, 2015]. It is fundamental in aging process of aerobic living organism by maintaining a high level of copy number of mitochondrial DNA [Li, 2015]. Interestingly, H$_2$S is the only species which is both substrate and inhibitor of the OXPHOS inside the mitochondria depending of its concentration [Szabo, 2014], which may to be in parallel to the famous observation that exogenous H$_2$S inhalation induce a suspended animation-like state in small mammals, known as artificial hibernation, or hypometabolism [Blackstone, 2005]. H$_2$S is known to protects against many cardiac conditions, including pressure overload-induced heart failure [Snijder, 2015]. This supports the hypothesis that endogenous H$_2$S is a regulator of energy production in mammalian cells particularly during stress conditions, which enables cells to cope with energy demand when oxygen supply is insufficient [Fu, 2012].

Moreover, we observed in fertilized chick's eggs an in-ovo anoxia reoxygenation. This study was performed by gently placing an H$_2$S microprobe above the ventricle of the chicken or the dorsal aorta. We observed that the H$_2$S level increased significatively within the anoxic (transient) cardioplegia (or when the heart beat extremely slowly) and decrease when it beat again. Therefore, it appears that the blood flow plays a role by removing endogenous production of H$_2$S which could bind deoxyhemoglobin to form sulfhemoglobin and propagate it.

Since many methods are well established to measure H2S in tissues [Olson, 2012], applying this approach to monitor the changes of metabolic activities induced by PBM is of high interest.

g) The level of hydrogen selenide (H$_2$Se)

Alongside oxygen and sulfur, selenium, the constitutive element of H$_2$Se belongs to the chalcogens group and have similar excretory and metabolic pathways. Analog to H$_2$S, H$_2$Se, is an endogenous small gaseous molecule which can induce a suspended animation like state and show reperfusion injury protection [Iwata, 2015]. It reversibly binds COX, which inhibits the mitochondrial respiration and argued to be the fourth gasotransmitors with H$_2$S, NO and carbon monoxide (CO) [Kuganesan, 2019]. Moreover, incorporated into numerous selenoprotein oxidoreductase enzymes as glutathione peroxidase, it is essential for maintaining redox-status homeostasis in health and diseases, and its deficiency induces a substantial increase of ROS, which is suspected to be one important cause of cancer and CVD [Bleys, 2008].

Since many methods are well established to measure H2Se in tissues or Selenium in serum, applying this approach to monitor the changes of metabolic activities induced by PBM is of high interest.

h) The concentration of ions.

Ions play an important role in the metabolism of all organisms as reflected by the wide variety of chemical reactions in which they take part [van Vliet, 2001]. Ions are cofactors of enzymes, catalyzing basic functions such as electron transport, redox reactions, and energy metabolism; and they also are essential for maintaining the osmotic pressure of cells. Because both ions limitation and ions overload delay growth and can cause cell death, ion homeostasis is of critical importance to all living organisms.

Since many methods are well established to measure ions in tissues (in particular calcium and potassium ions), applying this approach to monitor the changes of metabolic activities induced by PBM is of high interest.

i) The level of cytochrome, including cytochrome c oxidase, by NIRS.

It is well established that broadband Near InfraRed Spectroscopy (NIRS) can be used to monitor concentration changes of the oxidation state of cytochromes as cytochrome-c-oxidase ($\Delta$oxCCO) which plays a key role in the mitochondrial respiration [Roever, 2017].

Since different methods are well established to measure $\Delta$oxCCO in tissues (or other cytochromes involved in the metabolism), applying these methods, including NIRS to monitor the changes of metabolic activities induced by PBM is of high interest.

j) The use of medical (functional) imaging techniques (MRS "Magnetic Resonance Spectroscopy"; MRI "Magnetic Resonance Imaging"; NMR "Nuclear Magnetic Resonance"; PET "positron emission tomography"; EPR "Electron Paramagnetic Resonance"; SPECT "single photon emission computed tomography" ; BOLD "Blood oxygenation level dependent" NIRS "Near infrared Spectroscopy").

Metabolic imaging focuses and targets changes in metabolic pathways for the characterization of various clinical conditions. Most molecular imaging techniques are based on PET and MRS, including conventional $^1$H and $^{13}$C MRS at thermal equilibrium and hyperpolarized magnetic resonance imaging (HP MRI). The metabolic pathways that are altered in many pathological conditions and the corresponding probes and techniques used to study those alterations have been reviewed by Di Gialleonardo et al. [Di Gialleonardo, 2016]. In addition, Fuss et al. [Fuss, 2016] described the use of medical imaging to address various conditions in humans.

Since many metabolic imaging-based methods are well established to assess the metabolism, applying these methods, including functional metabolic imaging, to monitor the changes of metabolic activities induced by PBM is of high interest.

k) The vascular tone and the vasomotion.

Vascular tone refers to the degree of constriction experienced by a blood vessel relative to its maximally dilated state. All arterial and venous vessels under basal conditions present some degree of smooth muscle contraction between balance of constrictor and dilatator influences that determines the diameter of the vessel, e g the vascular resistance to adapt / regulate blood flow and pressure. Basal vascular tone differs among macro and micro-circulation and organs. Certain organs have a large vasodilatory capacity (e.g., myocardium, skeletal muscle, skin, splanchnic circulation) hence a high vascular tone, whereas others organs have relatively low vasodilatory capacity (e.g., cerebral and renal circulations), hence a low vascular tone.

The vascular tone regulation differs among the macro (arteries, veins) and the micro (arterioles, venules, capillaries). Notably, even if the tone can be modulated via extrinsic factor (nerves, circulating metabolites), blood vessel can exhibit spontaneous oscillations (vasomotion) which give rise to flow motion [Aalkaejer, 2011]. Therefore, through the dependence of the vascular tone in a multiplicity of actuator from local to systemic, the analysis of this tone, give insight on the metabolic activity, and can reflect the degree of aging [Bentov, 2015] and many pathophysiological conditions, as ulcer risk, type 2 diabetes [Smirnova,2013], endothelial dysfunction or hypertension [Ticcinelli, 2017], renal diseases [Loutzenhiser, 2002] [Carlstrom, 2015] or metabolic syndrome [Walther, 2015]. Moreover, the assessment of the skin microvascular endothelial function is used as diagnosis as well as prognostic of CVD [Hellman, 2015].

Since many methods are well established to assess to the vascular tone and the vasomotion, such as videocapillaroscopy, plethysmography [Tamura, 2019], laser doppler flowmetry, pressures measurement via cutaneous vascular conductance (CVC) or time frequency analysis as example, and since all the cardiovascular system is argued to be a single entity of coupled oscillators in a dynamic point of view [Shiogai, 2010], any methods which enable to assess to the change of metabolic activities induced by PBM (including heart rate variability (HRV) which give information on the autonomic nervous system via ECG or heart sound measurement [Alvarez, 2018] via phonocardiogram (PCG) [Patidar, 2014] are of high interest. As observe by the inventors along surgical procedure respiratory frequency variability (RFI) [stevanovska,2007] or ballistocardiography to monitor the changes of metabolic activities induced by PBM is of high interest.

1) The use of electromagnetic endogenous signals

Electrocardiogram (ECG), electroencephalogram (EEG) and electromyogram (EMG) are standard measurements of electrical activity of the metabolism of the heart, the brain and the muscle respectively. Novel ECG analysis based on signal computational classification [Patidar, 2015] are promised tools in heart diagnosis notably giving insight in coronary artery disease [Kumar, 2017] [Acharia, 2017], arrhythmia and ischemia disorders [Bhoi, 2017]. Same kind of analysis have been performed on EEG which show interesting outcomes in epileptic seizures, the most common brain disorders [Bhattacharyya, 2018].

Since many methods are well established to monitor these electromagnetic endogenous signals, methods which enable to assess to the change of metabolic activities induced by PBM are of high interest.

m) The use of bio impedance signals:

It can be used in the clinic for measuring various physiologic parameters [Petterson, 2016]. This approach is used for pacemakers as Ensite from St Jude Medical, OptiVol from Medtronic and closed loop stimulation from biotronik.

Since many methods are well established to measure electrical bioimpedance in tissue or directly on the skin, any these methods which enable to assess to the change of metabolic activities induced by PBM is oh high interest.

n) The presence of markers in the circulating blood.

A long list of circulating markers of interest to monitor the light dose during PDM includes metabolites (pyruvate, etc), coagulation factors, apoptotic factors, (pro and anti) inflammatory factors as well as hepatic factors, mitokines, or level of isolated mitochondria for instance. It should be noted that only a few of them is enumerate here.

Succinate: It is a key intermediate of the tricarboxylic acid cycle (TCA) cycle which plays an essential role in anabolic and catabolic pathways. It is notably associate within reperfusion injuries [Chouchani 2014]. Mitochondria are the physiological source of succinate, but accumulated succinate can be transported into the cytosol and then in the circulating blood. This TCA cycle intermediate connect intracellular metabolic status and intercellular signaling [Tretter, 2016]. Level of succinate in blood can varies from 2 to 20 $\mu$M, where this concentration can increase, within hypoxic stress, pro-inflammatory stimuli, exercise, or with pathological conditions such as type 2 diabetes, obesity or ischemia reperfusion injury [Grimolizzi, 2018].

Since circulating level of succinate can be monitored via bioluminescent assay, or Raman spectroscopy, then to assess to the change of metabolic activities induced by PBM via the circulating level of succinate is of high interest.

Lactate and lactate dehydrogenase (LDH): Since blood lactate is reduced within PBM during anaerobic exercise [Park, 2017] and since LDH is common marker of cell damage and cell death, using these markers to assess the change of metabolic activities induced by PBM is of high interest.

Moreover, combination of LDH level within aspartate aminotransferase (AST) level is a potent indicator. Note that lactate level can be assess with new aerometric method directly on the saliva [Tamura, 2018].

Level of circulating eNOS as well NO or nitrite or nitrate: the levels of these compounds are essential, notably for the regulation of systemic blood pressure and systemic homeostasis [Wood, 2013]. By extension, also levels of circulating $H_2S$ or sulfite or sulfate level can be assessed to monitor the change of the metabolic activities induced by PBM.

Since many pathologies are associated within a dysregulation of circulating glucose level, which directly induce systemic metabolic disorders, as it is the case for diabetes, monitoring the change of the metabolic activities through the assessment of the glucose level is of high interest.

Since it is recently shown that cell free functional mitochondria are present in the circulating blood and since it is also shown that mitokynes are important in the metabolic remodeling, especially in the heart failure [Duan, 2019] monitoring the change of the metabolic activities through the assessment of the circulating mitochondria level or mitokynes level is of high interest.

Cardiac markers: Several establish markers (myoglobin, creatine kinase isoenzyme, troponin I and T, B-type natriuretic peptide, transaminase) are clinically used for cardiac infarction diagnosis and also for other organs injuries. In a lesser extent, LDH, glycogen phosphorylase and recently ischemia-modified albumin can be used in diagnosis within 30-minute assay [Dasgupta, 2014]. This is also the case for thioredoxin level [Jekell, 2004].

Serum/plasma levels of thioredoxin: The level of this enzyme is elevated in infection, ischemia-reperfusion, and other oxidative stresses. Therefore, they are good markers for monitoring oxidative stresses. Plasma levels of thioredoxin are also elevated in patients with coronary spastic angina and other cardiovascular diseases [Nakamura, 2004].

Serum/plasma level of mtDNA copy number of leucocytes, total antioxidant capacity, or bicarbonate, malondialdehyde (MDA), uric acid, bilirubin but also cytokines or chemokine markers as IL6, IL10, or TNF$\alpha$ for instance level are also of high interest.

o) The use of voltage-sensitive fluorescent dyes for the optical mapping of cardiac electrical signals.

Although, the optical imaging of cardiac electrical signals using voltage-sensitive fluorochromes (VSF) has only been performed in experimental studies because these VSFs are not yet approved for clinical use, FDA approved dyes, such as Indo Cyanine Green (ICG) [Martisiene, 2016], exhibits voltage sensitivity in various tissues, thus raising hopes that electrical activity of cardiac tissues could be optically mapped in the clinic. Therefore, methods based on the use of voltage-sensitive dyes to map (or to assess locally with a "point measurement" system) the cardiac electrical signal to monitor/adapt the light dose during PBM is of high interest.

p) The use of redox sensors to assess of the status of various tissular redox states. Since metabolic and redox reactions are intricated and since many methods are based on the measurement of redox sensors proteins to asses to metabolic activities, monitoring changes of metabolic activities induced by PBM with redox indicators probes is of high interest.

q) The use of ultrasounds to assess the status of various tissues, including cardiac tissues.

Ultrasonography is a well-established method to investigate cardiac tissue. Many parameters characterizing the heart tissues as well as the blood flow are routinely obtained during ultrasonography.

Therefore, methods based on the use of ultrasonography to monitor the light dose during PBM are of high interest.

r) The use of the $pO_2$ (and / or the OCR) to reflect the metabolic activity of various tissue and / or of the whole body through the measurement of the partial pressure of arterial oxygen ($PaO_2$) and the fraction inspired oxygen ($FiO_2$).

$pO_2$ can be easily measured within exogenous or endogenous probes in different compartment (tissular or organ) or different organelles within the cell. For instance, such probes can be optically detected. Others techniques, such as EPR oximetry, polarographic electrodes or BOLD imaging are of high interest to assess changes of metabolic activities induced by PBM.

s) The use of hemodynamic variables.

In the clinic, real time assessment of these variables is a must to monitor the metabolic activity, especially in case of cardiovascular injuries. Such measurements notably involve, arterial and venous gases pressures, cardiac output, stroke volumes, capillary pressure, as well as systemic and pulmonary resistance. Therefore, the use of these methods to assess changes of metabolic activities induced by PBM are of high interest.

Optionally, the light power emitted by the device mentioned in page 8, or the light applied during the application method of PBM mentioned above, is to be combined with the administration of exogenous agents (see the list given below) to increase the PBM effects.

Indeed, the inventors demonstrated a synergy resulting from the combined administration of PBM with notably exogenous agents (sulfur donors) and nitric oxide donors.

As already shown by the inventors, an administration of ALA combined with PBM increases the PpIX build up and, consequently, the level of endogenous PpIX. Therefore, the coadministration of ALA and light is of high interest to increase PBM effects. It should be noted that other exogenous agents can be combined within PBM to increase its effects, as indicated below.

As presented in figure 3, the inventors recently observed that when PBM is combined with an FDA approved exogenous agent, sodium thiosulfate (STS), which are sulfur donors, the angiogenesis observed on the chick's Embryo Chorioallantoic Membrane (CAM) is even more stimulated. This strongly suggests that the combination of PBM with exogenous such agents stimulates even more angiogenesis, or the metabolic activity, than PBM or such agents applied separately.

The assessments of these PBM effects on angiogenesis were performed using an approach based on fluorescence angiographies performed on the CAM several days after PBM. Fig. 3 presents a typical CAM fluorescence angiogram (left image) characterized quantitatively with an image processing and analysis software developed in our laboratory [Nowak-Sliwinska, 2010]. The main objective of this development was to characterize dynamic changes taking place in the capillary/vessels network of the CAM between embryo development day (EDD) 6 and 12, when the CAM vessels are monitored using an epi-fluorescence microscope equipped with a scientific camera following the intravenous (iv) injection of a fluorescent agent [Nowak-Sliwinska, 2010]. From the resulting angiogram, 3 descriptors are extracted: the number of branching points/mm$^2$, the mean area of the vessels network meshes, and the mean of the 3$^{rd}$ quartile of the mesh area histogram. As presented in Figure 3, our proof of concept results demonstrates that PBM significantly stimulates the CAM angiogenesis. This effect is even more pronounced if PBM is combined with an exogenous application of 175 mM STS.

Interestingly, in parallel, we observed in-ovo, through the monitoring of the $H_2S$ and NO level on the chicken embryo, that a topical application of STS induced a significant increase of NO after a long time (6 at 12 hours), whereas, when performing a PBM irradiation 1 - 2 hours after the STS application, the time when NO was produced was significantly reduces, typically down to one hour.

Since STS is a clinically approved $H_2S$ donor [Snijder, 2015] to protects against many cardiac conditions, as also already reported for $H_2S$ [Yu, 2014], including pressure overload-induced heart failure via upregulation of endothelial nitric oxide (NO) synthase [Kondo, 2013] as well as renal ischemia / reperfusion injury [Bos, 2009], the combined use of PBM, applied with the device/protocol mentioned above, with the administration of $H_2S$ donors (such as STS or methylsulfonylmethane (MSM), or dithiolthiones for instance, or other donors presenting different $H_2S$ kinetics release) and/or NO donor substances, including NO itself, is of high interest.

Since Cysteine is an important source of sulfide in the human metabolism, combining the administration of this proteinogenic amino acid, or derivatives thereof, such as selenocysteine, or synthetic form as N-acetylcysteine is of high interest to potentiate the effects of PBM.

MSM, a naturally occurring organosulfur compound, is utilized as an alternative source of biologically active sulfur. It is mostly used for anti-inflammatory treatments. It has been investigated in animal models, as well as in many human clinical trials [Butawan, 2017]. MSM is also recognized for its antioxidant capacity and it was proposed that the antioxidant mechanism acts indirectly via the mitochondria rather than directly at the chemical level [Beilke, 1987]. As an FDA approved substance, MSM, is well-tolerated by most individuals at dosages of up to four grams daily, with very few side effects [Butawan, 2017]. Results from in vivo and in vitro studies indicate that MSM actions are at the crosstalk of oxidative stress and inflammation at the transcription and sub-cellular levels [Butawan, 2017]. Interestingly, Kim et al. [Kim, 2009] demonstrated that MSM can also diminish the expression of inducible nitric oxide (NO) synthase (iNOS) and cyclooxygenase-2 (COX-2) through suppression of the nuclear factor-kappa B (NF-κB), a transcription factor involved in the immune and cellular responses to stress. This observation is highly interesting since NO is a powerful vasodilator involved in many metabolic functions. As some other gas transmitters, called gasotransmitors [Donald, 2016], NO can have differential effects depending on its local concentration and microenvironment [Thomas, 2015] which can impact many different processes [Rapozzi, 2013; Reeves, 2009]. It has also been suggested that PBM causes NO photodissociation from COX [Karu, 2005; Lane, 2006]. Concomitantly, NO photodissociation from other intracellular "reservoirs" such as nitrosylated forms of myoglobin and hemoglobin have also been hypothesized [Lohr, 2009]. It is well established that cell respiration is down regulated by the NO production by mitochondrial NO synthase. The $O_2$ displacement from COX by NO inhibits cellular respiration, and ATP production [Antunes, 2004; Cooper, 2008]. Therefore, it is believed that PBM increases ATP production. An alternative and, possibly, parallel mechanism to explain the release and/or increase of NO bioavailability by PBM could be linked to an action of COX as a nitrite reductase enzyme (a one-electron reduction of nitrite gives NO), in particular when the $O_2$ partial pressure is low [Ball, 2011].

All together, these observations indicate that MSM has an indirect effect on the mitochondrial electron transport chain (ETC) through its NO modulation. In addition, this analysis of the literature indicates that the combined use of PBM with NO donors, including NO itself, induces a potent synergetic effect.

Moreover, since interaction between $H_2S$ and NO can produce nitroxyl (HNO), which plays an effective role within the cardiovascular system about oxidative stress and cardioprotection, heart contractility, vascular tone as well as angiogenesis [Nagpure, 2016; Wu 2018], the coadministration of (H)NO is of high interest to increase PBM effects.

Ebselen, an FDA approved $H_2Se$ donor is of high interest to increases PBM effects as already discuss by the inventor (page 13, point g).

As already mention, $NAD^+$ is required for redox reactions and control hundreds of key process of energy metabolism to cell survival, rising and falling depending on food uptake, exercise, and time of the days. Therefore, administration of $NAD^+$ donor, as vitamin B3 within PBM is of high of interest to increase PBM effect.

Curcumin, a major active component of turmeric (Curcuma longa, L.), is known to have various effects on both healthy and cancerous tissues. Notably, curcumin induces ER stress, thereby causing an unfolded protein response, the major retrograde signaling, and calcium release, which destabilizes the mitochondrial compartment and induce apoptosis.

By extension, any exogenous agents which are known to modulate the metabolism, especially within the mitochondria through the modulation of the ETC or ROS modulator for instance, are of high interest to increase PBM effects. It is the case for adenosine diphosphate (ADP) which is known to increase the OCR or following the administration of vitamin K or ketamine for instance, as well as bradykinin, catecholamines like adrenaline noradrenaline or dopamine, or opioid which activate various G proteins, or various kinases modulator or various anti-oxidant and/or anti-inflammatory donor as resveratrol for instance is of high of interest to increase PBM effect.

By extension, since temperature, exogenous or endogenous mechanical pressure [Li, 2005; Hwang 2003], physical exercise, hyperoxia, hemostasis (remote preconditioning) are known to modulate the metabolism, any exogenous stimulus, or combination of, like environmental/physical /electrical or electromagnetical stimulus applied on the biological object is of high of interest to increase PBM effect.

As observed by the inventors, the potency of PBM not only depends on the light dose [J/m$^2$] and spectroscopy (wavelengths) but, surprisingly also on the fluence rate. For example, the inventors have observed for a specific case, as indicated in figure 4, that cells must preferably be illuminated during 3 minutes with an irradiance (which is equal to the fluence rate in this specific setting consisting of cell cultures) of 3 mW/cm$^2$ (i.e. a dose of 0.54 J/cm$^2$). Different illumination times and/or irradiances do not induce any PBM effects (excepting "hot spots" observed as for instance with an irradiance of 15 mW/cm$^2$ applied for 40 seconds).

This is an illustration of the well-known bimodal effects of PBM, i.e. too high or too low fluence rates and/or light doses significantly reduce the PBM effects. The inventors have also demonstrated, in certain conditions, the absence of "neutralization" of the PBM effects by an overdose/irradiance before and/or after PBM applied with optimal conditions.

On the other hand, it is well established that the optical properties of biological tissues, described mostly by their absorption $\mu_a$ and reduced scattering $\mu_s'$ coefficients, have an important impact on the propagation of the light around a light distributor. In general, the fluence rate (and the light dose) decreases with the distance from the light source due to the absorption and scattering of the light in the tissue for a given power (and illumination time). Figure 5 illustrates how the fluence rate F (normalized by the irradiance E) decreases with the distance from the surface on the specific case of a "broad" (much larger than $\mu_{eff}^{-1}$), collimated illumination of a semi-infinite sample. Therefore, the fluence rate and the light dose in the tissues are never optimal at the same time in different locations of the tissues treated by PBM.

It should be noted that the geometry of the light distributor (illumination geometry) is adapted to the specific organs to be treated. For example, frontal (broad field), balloon-based, or interstitial illuminations, with one or several fibers, are considered, in particular (see the products commercialized by Medlight SA "http://www.medlight.com/#" as illustrative examples).

The inventors have also established a new method to adjust the radiometric and spectral conditions used in PBMT based on frequency analysis (in particular using the wavelet theory) of parameters reflecting the metabolic activity. More precisely, they have conducted a time frequency analysis of the partial pressure of oxygen ($pO_2$) of the chick's embryo chorioallantoic membrane (CAM) during PBM.

It is well known that arterioles, particularly in the peripheral microcirculation, strongly respond to the surrounding tissue $pO_2$ [Jackson, 2016] through complex metabolic regulation mechanisms [Reglin, 2014] where low frequency oscillations of the blood perfusion exist [Kvandal, 2006]. Based on local measurements of the $pO_2$ performed in the CAM during a "long" (several minutes) time using commercially available Clark's probes (Unisense®, OX-needle, OX100-Fast) we calculated frequency spectra resulting from a wavelet-based analysis of the $pO_2$. Wavelet analysis is a well-known mathematical transform which enables to characterize nonstationary frequencies during the measurement time. Figure 6 (middle left) shows a typical measurement of the $pO_2$ close to a new CAM arteriole at embryo development day (EDD) 7. Here, the time signal (measurement time: ~180 s) mostly results from the superposition of 2 frequencies (see figure 6, lower left; the vertical axis is the frequency): the heartbeat (~1 Hz) and the myogenic tone (~0,1 Hz) that represents the intrinsic activity of the vascular smooth muscle.

$H_2S$ is a potent regulator of the vascular tone [Köhn, 2012] which can be induced by the administration of NaSH. We measured with our Clark's probe that a $H_2S$ stimulation of a CAM arteriole induced by the topical application of NaSH (10 $\mu$l, 1 $\mu$M in physiologic serum) generates a strong modulation of the $pO_2$ around 60 mmH. This modulation is observed at least for the myogenic (0,05Hz - 0,15Hz), the endothelial nitric oxide synthase dependent (0,01Hz - 0,02Hz) and the endothelial nitric oxide synthase independent (0,005Hz - 0,01Hz) bands. Other lower frequencies bands are also activated where it was notably suggesting that some of them are correlated within prostaglandin or prostacyclin release from the endothelium.

Our innovation results from PBM irradiations of the CAM we have performed with a frontal light distributor (850 nm, 7 mW.cm$^{-2}$, 30 s) at t=80 min and t = 105 min (see figures 7). A modulation ("dimming") of these frequencies (figure 6 (top right) and figure 7) is clearly induced by PBM.

It appears, in particular, that the bands 3, 5 and 6, as well as those corresponding to undefined lower frequencies, are fully or partially inhibited by PBM (see figure 6). Inhibition of band 3 (the myogenic one) indicates that, since this band is due to arteriolar smooth muscle cells via activation of NADPH oxidase (NOX) and subsequent ROS generation [Nowicki, 2001; Li, 2017], PBM inhibits the NOX activity. Since the NOX superfamily plays a fundamental role in inflammatory and immune responses where notably NOX are implicated in the metabolic switch of leucocytes activation our observations reveal an important pathway of PBM to modulate inflammations and be potent as an important immunomodulator. Moreover, modulate the myogenic tone of the biological object by PBM can prevent numerous hypertension injuries as it is the case for instance in renal pathologies [Loutzenhiser, 2002; Moss, 2016]. Altogether, our results indicate that a frequency analysis of a parameter reflecting the metabolic activity, such as the $pO_2$, enables to monitor the light dose and/or the fluence rate used for PBM. It should be noted that the oscillations observed in figures 6 and 7 were induced by $H_2S$ for illustrative purpose, i.e. to increase the signal to noise ratio. Indeed, such oscillations are present even in the absence of H2S.

Therefore, adapting the radiometric and spectral conditions used in PBM therapy based on the frequency analysis of parameters reflecting the metabolic activity is of high interest.

This specific type of monitoring can be performed for two main purposes: i) to apply the PBM light at an optimal time relative to the metabolic "oscillations" and, ii) to assess the level of change of the metabolism induced by PBM in such a way that it is optimal.

The $pO_2$, as presented just above, is not the only parameter to be analyzed using the wavelets, or frequency-based analysis to monitor the light dosimetry during PBM. The list presented above in pages 9 to 19 (List of feedback observables a to s) describes other parameters of interest:

The inventors have also shown that application(s) time of light irradiation within the biological object is crucial to induce significant PBM effects. These observations are very important since biological objects are dynamic within a wide frequency scale of metabolic activity triggered from transient or regular endogenous or exogenous factors. Notably, the inventors have shown that, when light is applied at a specific time during the metabolic activity of glioma cells or HCM, the metabolic response of cells is significatively modulate differently which modulate accordingly phenotypical long-time response. Inventors have also shown, using the in-ovo chicken embryo heart models during anoxia / reoxygenation studies, that the survival rate is significantly higher when the PBM irradiations start just before the reoxygenation, compare to when PBM is performed during ischemia long time before or long time after the reoxygenation. Moreover, the inventors have shown that PBM restart or modulate the heartbeat following an ischemic cardioplegia or which present bradychardia or tachycardia, whereas no influence is observed on healthy beating hearts.

The inventors have also shown that PBM can be used for the preconditioning of the heart chicken embryo during hypoxia reoxygenation events.

One aspect of the present invention is the application of the device or method to treat ischemia reperfusion injury, in particular those affecting the myocardium to reduce the infarction size following acute myocardium infarction (MI).

Based on the chicken embryo heart the inventors developed an anoxia reoxygenation experiment in ovo where eggs were placed in a thermoregulated gas chamber with a continuous monitoring of the environmental and embryonic temperature and $pO_2$. For some experiments, small $H_2S$, NO and pH probes were also positioned around the embryonic heart or at different location of the embryonic tissue. This chamber was placed under a microscope for image recording. After a stabilization time (temperature stabilization), an anoxic environment was created by flushing nitrogen all around the egg during tens of minutes without any change of the environmental temperature, followed by a reoxygenation of the egg as depicted on the figure 8.

At Embryonic Development Day (EDD) 3, flushing pure $N_2$ during 45 min before a reoxygenating of the embryos induced a mortality rate larger than 50 % 48h after the end of the experiment. This experiment supports one aspect of the "reperfusion injury" mentioned as the "oxygen paradox" in the article published by Latham et al. (Latham, 1951), i.e. that reperfusions could be, in certain cases, lethal (Piper, 2000). In our case, as well for isolated chicken heart embryo (Raddatz, 2010), reoxygenation induce a burst of Reactive Oxygen Species (ROS) and a permanent or transient cardioplegia followed by irregular heartbeat (bradycardia, tachycardia). In our experiment, when embryo at EDD3 undergo a 45 min anoxia, we observed that a photoconditioning (671 or 808 nm, 5 mW.cm$^2$, 30 s) of the embryo just before the reperfusion significantly avoid cardioplegia and, importantly, increase the survival rate at 48 h. Interestingly, this positive effect of PBM is not observed if light is applied too early or too late after the reoxygenation. This last observation clearly suggests that the time at which the light is applied relative to the reoxygenation is critical to produce a beneficial outcome.

In conclusion, one application of high interest for the invention consists to use PBM delivered by our original medical device and method to treat damages resulting from hypoxia reoxygenation events and by extension for ischemia reperfusion events.

The inventors have also shown that the heart beat can be stimulated by PBM after an anoxic non-permanent cardioplegia of the chicken embryo heart.

Before Embryonic Development Day (EDD) 7, oxygen supply of the chick's embryo was mainly performed by diffusion across the shell, then through the embryo. Heart beat and blood flow, which are observable from EDD 2, mainly act as stimulus for cardiovascular development. Embryo, up to day 5 are flattened on the "surface" located just below an albumin layer. Therefore, it is easily accessible after removing a part of the shell. This is why, in parallel to the chicken embryo ontogeny, embryo from EDD 2 up to EDD 5 are used since decades as excellent models for developmental biology and, in particular, in cardiogenesis and rythmogenesis. This model is also used for anoxia-reoxygenation studies [Sedmera, 2002] where their behaviors are studied during and following hypoxia or anoxia, but also during hypoxic induced tachycardia, bradycardia or for fibrillation studies.

One interesting PBM effects observed by the inventors during an anoxic in ovo experiment is in relation with the positive effect(s) of light which enable to restart the heard after a cardioplegia. Indeed, a prolonged anoxia leads to a stop of the heart beat which, sometimes, restarts to beat again transiently until an irreversible and total cardioplegia takes place. The inventors observed, in most cases, that a PBM irradiation often restart the beating heart (Figure 9) after such a cardioplegia.

This surperising positive effect of PBM strongly suggests that it triggers the metabolic activities involved in the heart beat, including after an anoxic cardioplegia. Since PBM is known to reduce inflammations and to boost the metabolic activity, PBM is of high interest to treat conditions such as fibrillations, including atrial fibrillations, for instance

By extension, since the metabolism is subject to autonomous (i.e. independent of the cell cycle [Papagiannakis, 2017]) and non-autonomous rhythms of various frequencies, as it is the case, for instance, for the circadian rhythm [Bailey, 2014], applying PBM light at specific times and/or frequencies to lock, trig and/or (re)synchronize metabolic oscillations is of high of interest, in particular to treat various metabolic disorders, such as type 2 diabetes [Petrenko, 2020], metabolic switch as aerobic glycolysis (Warbugg effect) in cancer cells [Gatenby, 2018], or within hepatic disorders and diseases [Zhong, 2018].

**Detailed description of the invention**

According to the results presented in Figure 4 that shows PBM effects on the ability of HCM cells to produce PpIX, a fluence rate (or intensity) of 3 mW/cm$^2$ must be applied during 3 minutes to maximize this effect. Since the fluence rate is not uniform in bulk (or 3D) tissues for a fixed irradiance, as illustrated in figure 5 for a particular geometry and specific optical coefficients, the medical device according to the invention preferably delivers an irradiance which changes with time in such a way that all cells receive the optimal fluence rate during 3 minutes. In most situations, the illumination geometry is not changed during the illumination. Therefore, since the irradiance is given by the power [W] illuminating the tissues divided by the surface [m$^2$] of the illumination spot.

Let's consider the specific situation corresponding to the geometry and the optical coefficients mentioned in figure 5. Since the fluence rate F can be determined by the solution of the diffusion approximation (nb: the hypothesis that are at the basis of this diffusion approximation must be fulfilled, i.e.: i) $\mu_a << \mu_s'$; and ii) we are looking at the fluence rate at locations "z" which are "far" (i.e. $z > 1/\mu_s'$) from the light source(s) and boundaries)

$$F = k.E.e^{-\mu eff\,z} \qquad\qquad \text{Equation 1)}$$

, the only way to maintain F constant, called F' thereafter, for increasing values of z is to increase E. This statement derives from the inversion of the previous expression which writes:

$$E = (F'/k).\,e^{\mu eff\,z} \qquad\qquad \text{Equation 2)}$$

Since each HCM cell must be illuminated during 3 minutes with F' = 3 mW/cm$^2$, another concept of important has to do with the tolerance affecting this fluence rate. If cells must be irradiated with exactly 3 mW/cm$^2$ the total treatment of the whole sample would take an infinite time since the volume corresponding to these cells is equal to 0 mm$^3$ (they are confined in a plane located at depth "z" which has a volume equal to 0 mm$^3$). However, looking at figure 4 indicates that the irradiance full width half maximum (FWHM) of the "peak" located at 3 mW/cm$^2$ and 180 s is about 3.2 mW/cm$^2$, i.e. the fluence rate F' must range within 3 ± 1.6 mW/cm$^2$ (written F'±$\Delta$F' thereafter) to induce optimal PBM effects after 180 s. It means that the HCM cells in which an optimal PBM effect is induced are not in a plane but in a slice of thickness $\Delta$z. The thickness $\Delta$z of this slice is given by:

$$\Delta F = \Delta F'/(dF/dz) = \Delta F'/\,k.E.\,\mu_{eff}\,.e^{-\mu_{eff}\,Z}. \qquad\qquad \text{Equation 3)}$$

Since E = (F'/k). e$^{\mu effz}$, we have

$$\Delta z = \Delta F'/\mu_{eff}.F'. \qquad\qquad \text{Equation 4)}$$

Therefore, $\Delta$z only depends on F', $\Delta$F' and $\mu_{eff}$.

If the tissue volume to be treated ranges between zi(proximal position) and $z_2$ (distal position), the number "n" of different irradiances to apply during 180 s (thereafter called T) is equal to: $z_2$ - $z_1$ /$\Delta$z.

Consequently, the spatial evolution of the irradiance E is as presented in figure 10.

The temporal evolution of the irradiance E(t) is (see figure 11):

$$E(t) = (F'/k).\,e^{\mu eff.\ \Delta z.t/T} = (F'/k).\,e^{\Delta F'.t/F'.T} \qquad\qquad \text{Equation 5)}$$

where E will be equal (providing that the diffusion approximation equation is valid) to F'/k.e$^{\Delta F'/2F'}$ when 0<t<T, F'/k.e$^{3\Delta F'/2F'}$ when T<t<2T, F'/k.e$^{5AF'/2F'}$ when 2T<t<3T, F'/k.e$^{(2n+1)\Delta F'/2F'}$ when nT<t<(n+1)T, with n = $\mu_{eff}$.F'.($z_2$ - $z_1$)/$\Delta$F' (see equation 6 below).

Therefore, the total time "t$_{tot}$" it takes to treat a volume of tissue ranging between zi and $z_2$ is: T.($z_2$ - zi)/ $\Delta$z. With the explicit expression of $\Delta$z (Equation 4) we have:

$$t_{tot} = T(z_2 - z_1)/(\Delta F'/\mu_{eff}.F') = T.\mu_{eff}.F'.(z_2 - z_1)/\Delta F'. \qquad\qquad \text{Equation 6)}$$

Finally, it should be noted that, similarly to F', T can be applied with a certain tolerance due to the FWHM of the PBM peak along the illumination time axis (see figure 4).

In summary, in this example the device according to the invention applies an irradiance E(t), during a time which ranges between 0 and $t_{tot}$, given by: $E(t) = (F'/k) \cdot e^{\Delta F' \cdot t/F' \cdot T}$

Since the device illuminates a certain area of surface S [$m^2$] with a certain power P [W], we have that $E(t) = P(t)/S$.

Therefore, the device delivers an optical power P(t), during a time which ranges between 0 and $t_{tot}$, given by:

$$P(t) = (S.F'/k) \cdot e^{\Delta F' \cdot t/F' \cdot T} . \qquad \text{Equation 7)}$$

All parameters involved in the expressions of E(t) and $t_{tot}$ are determined for a specific organ (of known thickness $z_2$ - zi) and illumination geometry (of surface S): Indeed, F', $\Delta F'$ and T are derived from the figure 4 (for HCM), whereas k is derived from the tissue optical coefficients (which are known for the tissue of interest).

Example 1: Treatment of ischemia-reperfusion of heart muscle

A detailed example of the device according to the invention is presented in this example.

This treatment of ischemia-reperfusion of heart muscle can be performed:

1. During acute or chronic myocardial infarction (MI).
2. During aortic clamping and cardioplegia induced by post extracorporeal circulation.
3. During organ transplants (heart, lung or other).

The optical distribution routes considered are:

1. Interstitial (trans-myocardial)
2. Endocavitary (endoventricular)
3. Endovascular (endocoronary)

**Trans-myocardial medical device**

This involves implanting light distributors, preferably cylindrical and based on one or more optical fibers, through the heart (Figure 12 and 13) by a cardiac surgeon during myocardial revascularization consecutive to the acute phase of a MI or following an aortic clamping of more than 120 minutes under extracorporeal circulation, or during a heart transplant.

These light distributors are placed in the suffering cardiac area (ischemic area for example) at the end of the surgical procedure before reperfusing the coronary arteries.

These light distributors are placed according to the procedure described below:

1) Localization and estimation of the area to be treated by:

a. Macroscopic assessment of the area suffering (Figure 14) (visual indicators: edema, akinesia, dyskinesia, vermilion color chromatic appearance and / or use of characterization apparatus)
b. Correlation to the coronarography and ultrasound data corresponding to the ischemic myocardial anatomical area (for example for the left ventricle):

i. Anterior territory
ii. Lateral territory
iii. Inferior territory

2) Determination of the number of light distributors and their relative location necessary for optical delivery on the basis of the extension and accessibility of the ischemic area:

a. In situ consideration of the distribution of the coronary arteries to avoid transfixing them
b. The light distributors are transfixing and implanted at angles ranging between horizantal to normal to the surface, in the thickness of the myocardium (according to ultrasound data in time-movement mode) both to maximize the volume to be treated by distributors and allow their fixation as close as possible to the epicardium

c. The light distributors have a length (5cm) greater than their maximum length in the myocardium to distribute the light throughout the myocardium thickness.

3) Placement and fixation of a semi-rigid and transparent silicone-type or biodegradable mask by 4 points (single-strand 5.0 wire) on the epicardial surface of the heart to be treated. This mask serves both as a guide / template for the transfixion (pre-drilled at the correct implantation angles and whose holes follow geometric patterns and predefined spacings taking into account the propagation of light in the tissue depending on the light wavelength(s) used). This mask is also used to maintain the transfixion catheters into which the light distributors are inserted.

4) Transfixion of the myocardial wall through the mask with, for instance, a iCAT® type catheters whose characteristics are:

a. less than 20 gauges (to minimize hemorrhages and tissue damage),
b. hollow,
c. transparent at the used wavelengths,
d. closed end cap,
e. visual markings on the catheter indicating the length of catheter placement.

5) Connection to the light distributor device followed by an optical calibration step

6) Introduction of a light distributor into each catheter (similar to the interstitial procedure used in TOOKAD®-type photodynamic therapy). Attaching the distributors to the catheter via a luer lock. Attachment of all light distributors to the mask and / or to the patient's skin.

7) Determination of the temporal evolution of the light power emitted by the light distributors so that the treated cells receive, for an optimal time, a spatial irradiance ("Fluence rate") which is also optimal. This determination is made on the basis of the number and positioning of the light distributors determined by the user. It consists, for example, in deriving the temporal evolution of the light power emitted by the light distributors from the preset weighting matrix. These matrices are generated on the basis of a Monte-Carlo type simulation of the propagation of light in the tissues of interest and cost reduction algorithms taking into account the specificities (optical and biological) of each wavelength used to optimize processing time.

Optical delivery in such a way that the time evolution of the light power of the light source is done according to the determination described in the previous step. It can be modulated by monitoring methods based on various instrumental or biological data previously described. A simplified diagram illustrating an example of a part of the device supplying an optical distributor is presented in figure 15.

In the case of a single irradiation:

i. Surgical reperfusion procedure
ii. Removal of catheters and mask
iii. Hemostasis if necessary, at transfixion points
iv. Validation of the calibration of light distributors
v. Continuation of the surgical intervention as usual.
vi. Normal closing procedure as for any sternotomy.

In the case of multiple irradiations:

i. Surgical reperfusion procedure
ii. The catheters and light distributors are let in place and attached to the biodegradable mask. The light distributors can be tunneled to the skin, fixed and let in place for 8 to 10 days in order to repeat the procedure remotely.
iii. Continuation of the surgical intervention as usual.
iv. Normal closing procedure as for any sternotomy.
v. The removal of the light distributors is done with chest drain in place, by a simple manual removal with an ultrasound check at the second hour.

**Endoventricular medical device**

This involves the placement of one or more light distributors percutaneously into the left ventricle by an interventional cardiologist under fluoroscopy during myocardial revascularization in the acute phase of a MI in pre, per or post conditioning.

These light distributors are placed at the start of the procedure before revascularizing the occluded coronary artery(ies).

These light distributors are implanted according to the procedure described below:

1) Access to the radial or femoral artery by ultrasound puncture.
2) Introduction of a Radiofocus-type guiding sheath (5F-6F), using the Seldinger method.
3) Under fluoroscopy, navigation with a Radiofocus-type diagnostic guide catheter to the left ventricle after crossing the aortic valve. Removal of the guide and placement, via the diagnostic catheter of the light distributor with or without a self-inflating systolo-diastolic balloon in the left ventricle, or directly fixed in the wall of the ischemic left ventricle.
4) Optical delivery. The determination of the temporal evolution of the light power emitted by the light distributors and the optical delivery is performed as described above.
5) Removal of the catheter, light distributors and sheath; possibility to let the device in place for 8 to 10 days.
6) Usual procedure for reperfusion by interventional radiology of the occluded coronary arteries.

**Intravascular medical device**

This involves the placement of one or more light distributors percutaneously in the arteries, whatever they may be, under fluoroscopy during a revascularization process after an ischemic phenomenon in interventional radiology, in pre, per or post conditioning, during a MI, a lung or other organ transplantations submitted to ischemia reperfusion phenomena.

These light distributors are placed at the beginning or at the end of the procedure before reperfusion.

These light distributors, in the case of the coronary arteries, are implanted according to the procedure described below:

1) Radial or femoral arterial access with I.V catheter (Surflo® type).
2) Introduction of a Radiofocus-type sheath (5F-6F), using the Seldinger method.
3) Under fluoroscopy, navigation with a diagnostic catheter (4F, type JR4,) and a 0.035" guide (Radiofocus type), up to the coronary artery.
4) Removal of the guide and placement, via the diagnostic catheter, of the light distributor.
5) Optical delivery. The determination of the temporal evolution of the light power emitted by the light distributors and the optical delivery are performed as described above.
6) Removal of the catheter, light distributors and sheath.
7) Procedure for reperfusion of the coronary artery as usual.

Example 2 : continuous temporal change of the irradiance (or power) emitted by the light source.

Since $\Delta F'$ is larger than zero, the temporal evolution of the irradiance (or power) delivered by the light source can be continuous instead of incremental (as presented by the dotted lines fitting the histograms in figure 10 and 11). In this case, the temporal evolutions of the irradiance or the power delivered by the light distributor(s) is given by equations 5 and 7, respectively.

Example 3: temporal decrease, instead of an increase, of the irradiance (or power) emitted by the light source.

The different tissue layers of thickness $\Delta z$ can be illuminated with the appropriated fluence rate while increasing or decreasing (incrementally and/or continuously) the irradiance (or the power). In this case, $t_{tot}$ is the same, but the temporal evolution of the irradiance (or the power), are given by the expression $E(t) = (F'/k).e^{\Delta F'.(ttot-t)/F'.T}$ or $P(t) = (S.F'/k).e^{\Delta F'.(ttot-t)/F'.T}$ respectively ($0<t< ttot$).

Example 4: different illumination (light delivery) geometries.

Figure 5 shows the spatial distribution of the fluence rate for a specific geometry, namely in a semi-infinite tissue illuminated with a broad, collimated and perpendicular light beam at the air-tissue interface. It is well known in the field of photomedicine, in particular in photobiomodulation or LLLT, that different illumination geometries are considered depending on the tissue/organ structure(s) and access. Figure 6 illustrates some of the most common illumination geometries.

Solutions of the diffusion approximation exist for many of these geometries to determine the fluence rate. Therefore, a general expression of equations 5 for other illuminations, organs and/or light delivery geometries can be written as: $E(t) = F_E(\mu_a, \mu_s, g, n_{ext}, n_{tissue}, F', \Delta F', S, T, t)$, where $F_E$ is a function which depends on the tissue optical parameters, the organ and illumination geometries, the fluence rate, as well as its FWHM, and the illumination time(s) generating local maxima of the PBM effects. Numerous different approaches are known to determine $\mathbf{F}_E$, as described below in example 6.

Similarly, a general expression of equations 7 for other illuminations, organs and/or light delivery geometries can be written as: P(t) = $\mathbf{F}_P(\mu_a, \mu_s, g, n_{ext}, n_{tissue}, F', \Delta F', S, T, t)$, where $\mathbf{F}_P$ is a function which depends on the tissue optical parameters, the organ and illumination geometries, the fluence rate, as well as its FWHM, and the illumination time(s) generating local maxima of the PBM effects. Numerous different approaches are known to determine $\mathbf{F}_P$, as described below in example 6.

A combination of the light delivery geometries presented in figure 16 may also be used. Obviously, use of optical sources, as LED or VCSEL *exsitu* or *insitu* for instance, in direct contact or in quasi contact must also be envisaged.

Finally, heterogeneous tissues, in particular layered tissues structures, must also be envisaged.

Example 5: different tissue optical properties.

Since different types of tissues have different optical properties, the formalism described above is valid for different values of $\mu_a, \mu_s, g, n_{ext}, n_{tissue}$, in particular if these optical properties are subject to changes for a given tissue during the illumination.

Example 6: Assessment of the fluence rate using other approaches but those based on the diffusion approximation.

Different approaches are well established to model the propagation of light in biological tissues [Martelli, 2009]. Therefore, these approaches can be used instead of, or in combination with, the formalism presented above, which is based on the diffusion approximation of the light transport equation, to determine the temporal evolution of the light source to generate optimal PBM effects. These approaches, which have been mostly developed in photomedicine to master the dosimetry of light in tissues, are classified in two categories:

1) The analytical approaches: Excepting the well-known diffusion approximation of the light transport theory which was used to establish the formalism presented above in the detailed description of the invention, other analytical approaches are well established in this field, including but not limited to: The Kubelka-Munk theory, delta-Eddington radiative transfer equation, ...

2) The computer-based approaches: numerous computer-based approaches have been proposed since decades to simulate the propagation of light in biological tissues. These approaches include, but are not limited to: Monte-Carlo simulations, Finite elements simulations, ...

Example 7: The taking into account of the different tissue responses to PBM depending on the cell types, wavelengths (spectral design) and metabolic activities.

The specific values given above for F'(3 mW/cm$^2$), $\Delta F'$(1.6 mW/cm$^2$) and T (180 s) result from our experimental observations obtained in specific conditions in terms of sample (human cardiomyocytes: HCM), environment (medium, temperatures, pO$_2$, ...) and spectral design (only one illumination performed at 689 nm). However, changing one, or a combination, of these conditions would lead to different values for F', $\Delta F'$ and T, in particular. This is, in particular, the case if the chronogram of the application(s) of light is changed.

Therefore, the concepts presented above may be generalized for different conditions and cell types.

Example 8: Illumination of the tissues with radiometric conditions (irradiance/fluence rate, duration/dose) corresponding to multiple "hot spots" in figure 4.

It should be underlined that figure 4 presents several "hot spots". Two "hot spots", namely the one, mentioned above, which corresponds to an irradiance of 3 mW/cm$^2$ and an illumination time of 180 s (hot spot 1), and a second one at 15 mW/cm$^2$ and 40 s for the irradiance and the illumination time, respectively (hot spot 2).

This is important, in particular to minimize the total treatment time.

Indeed, since "high" irradiances cannot be applied without damaging the tissues, only tissues located "close" to the illumination surface can be treated with the "high" irradiance of 15 mW/cm$^2$. Otherwise, cells close to the light source would experience thermal damages when distant cells receive a relatively high fluence rate.

It is well accepted by the scientific community of this field that thermal effects start to be significant if an irradiance of several hundreds of mW/cm$^2$ of red (or NIR) light is applied during more than several seconds over a broad (diameter larger than $\mu_{eff}^{-1}$) spot.

Figure 17 and 18 below illustrate how to take profit of the presence of the "hot spot 2" to minimize the treatment time if the irradiance must be less than 100 mW/cm$^2$ (the other conditions are identical to those considered for figures 10 and 11).

In this case, the treatment algorithm looks as follows:

As long as E < 100 mW/cm$^2$, the temporal evolution of this irradiance (or power) is given by the equations 5 (or 7) with: F' = 15 mW/cm$^2$; $\Delta$F' = 4 mW/cm$^2$ and T = 40 s. Otherwise, the values of F' = 3 mW/cm$^2$; $\Delta$F' = 1.6 mW/cm$^2$ and T = 180 s must be used.

This example can be enlarged by the use of the combination of wavelength(s) within their own hot spot(s).

Example 9: Use of a passive attenuator to generate a temporal evolution of the irradiance (or power) with continuous wave (CW) light sources according to equations 5 and 7.

Numerous light sources are commercially available nowadays to treat tissues by PBM. Needless to mention that none of them generate an irradiance (or power) according to equations 5 (or 7). However, since many of these commercially available light sources emit CW light and generate an irradiance larger than 0.62 mW/cm$^2$ (3 mW/cm$^2$ divided by k = 4.87 in our specific conditions), they may be combined with an attenuator which would change its transmission with time in such a way that the irradiance would correspond to the value given by equation 5.

More precisely, if E' is the irradiance produced at 689 nm by such a source without attenuator, the temporal evolution of the attenuator transmission ($T_r$) would be given by: $T_r(t) = E(t)/E'$, where E(t) would be given by equation 5.

In summary, a particular design of the device according to the invention must integrate CW light sources combined with one or several attenuators to end up with an irradiance corresponding to that given in equation 5.

The generalizations mentioned above in Examples 1 to 8 also apply to this example.

Example 10: Use of high frequency modulation of light superposed to the temporal evolution of the irradiance or power given in example 4.

Since biological objects have dynamic optical absorption and responses to light, in part due to dynamic changes of their redox states, wavelength or multiplexed wavelengths used for PBM can be synchronized/modulated at higher frequencies than the temporal variation defining in equation 5 taking into account the kinetics/dynamic of the oxidative metabolic redox states.

Example 11: Use of pulse modulation to generate the temporal the irradiance (or power) with pulsed light sources according to equations 5 and 7.

As already mention in example 10, light can be modulated at higher frequency than the apparent frequency induces by the temporal evolution of the optical power. Then, P(t) can be seen as the time average of a pulsed optical power p(t), where:

$$P(t) = \int p(t)dt$$

Temporal evolution of P(t) can be induce by the modulation of the duty cycle of p(t).

Example 12: Combined use of local and/or systemic illuminations.

Since PBM induces, in certain conditions, bystander or abscopal effects, illuminating simultaneously or sequentially different parts of the biological object, including circulating objects such as the blood, is of high interest.

Example 13: Treatment by PBM of circulating biologic object into blood or lymphatic vessels.

1) A temporal variation of the light power or irradiance can be performed to illuminate, within a range of fluence rates, circulating biologic object passing in the vicinity of the light distributor. The expressions of the irradiance and power given in example 4 are adapted to take into account the different speeds of the biologic object into blood or lymphatic vessels targeted by PBM.

2) The power of light is synchronized with hemodynamic variables, such as the changes of flow due to the heart beat and the vasomotion, to irradiate optimally the targeted biologic object within the blood flow.

For instance, in order to overpass negative outcomes of the SARS-CoV-2 and, more generally, in the case of ARDS, optimizing the immune response, the hemoglobin oxygen affinity, the thrombogenesis processes and to promote the tissue regeneration using notably the bystander effects of PBM by inserting one or several light distributors in blood vessels, such as lung arteries, has shown impressive positive effects, as demonstrated by the inventors.

In particular, an example of a clinical procedure to position light distributors in the right and left pulmonary arteries is described below:

1. Venous access by puncture of the right jugular vein under ultrasound imaging using an I.V catheter (Surflo, Terumo).
2. Introduction of a 7 Fr sheath (Radiofocus, Terumo) using the Seldinger method.
3. Under fluoroscopic guidance, use a 4 Fr JR 4 guiding catheter (Cordis) in order to engage the ostium of the right pulmonary artery, using a non-hydrophilic 0.035-inch guidewire (Radiofocus, Terumo).
4. Removal of the 0.035-inch guidewire and connection of a hemostasis valve Y connector to the 4 Fr JR 4 guiding catheter.
5. Placement of the optical distributor through the 4 Fr JR 4 guiding catheter between the entry of the upper and lower right lobar pulmonary artery.
6. Total removal of the 4 Fr JR 4 guiding catheter from the 7 Fr sheath.
7. Flush the 4 Fr JR 4 guiding catheter with saline through the hemostasis valve Y connector.
8. Use the same procedure as described in 3, 4, 5 and 6 with a second 4 Fr JR 4 guiding catheter in order to place a second optical distributor between the entry of the upper and lower left lobar pulmonary artery.
9. Attachment of the 2 optical distributors (intubated in the 4 Fr JR 4 guiding catheter) to the skin with an adhesive system (Grip Lock, Vygon).

**References**

Aalkjær, C., Boedtkjer, D. & Matchkov, V., 2011. Vasomotion - what is currently thought? Acta Physiologica, 202(3), pp.253-269.

Acharya, U.R. et al., 2017. Application of higher-order spectra for the characterization of Coronary artery disease using electrocardiogram signals. Biomedical Signal Processing and Control, 31, pp.31-43. Available at: http://dx.doi.org/10.1016/j.bspc.2016.07.003

B. Alberts, A. Johnson, J. Lewis, M. Raff, K. Roberts, and P. Walter, Molecular Biology of the Cell, 4th ed. Garland Science, 2002.

Álvarez, R.C. et al., 2018. Cardiac Hemodynamic Monitoring in the Subcutaneous Space: A pre-clinical proof-of-concept. MeMeA 2018 - 2018 IEEE International Symposium on Medical Measurements and Applications, Proceedings.

F. Antunes, A. Boveris, and E. Cadenas, "On the mechanism and biology of cytochrome oxidase inhibition by nitric oxide," Proc. Natl. Acad. Sci. U. S. A., vol. 101, no. 48, pp. 16774-16779, Nov. 2004.

Bailey, S.M., Udoh, U.S. & Young, M.E., 2014. Circadian regulation of metabolism. Journal of Endocrinology, 222(2). DOI: https://doi.org/10.1530/JOE-14-0200.

K. A. Ball, P. R. Castello, and R. O. Poyton, "Low intensity light stimulates nitrite-dependent nitric oxide synthesis but not oxygen consumption by cytochrome c oxidase: Implications for phototherapy," J. Photochem. Photobiol. B, vol. 102, no. 3, pp. 182-191, Mar. 2011.

S. Banerjee and D. K. Bhatt, "Histochemical studies on the distribution of certain dehydrogenases in squamous cell carcinoma of cheek," Indian J. Cancer, vol. 26, no. 1, pp. 21-30, Mar. 1989.

M. A. Beilke, C. Collins-Lech, and P. G. Sohnle, "Effects of dimethyl sulfoxide on the oxidative function of human neutrophils," J. Lab. Clin. Med., vol. 110, no. 1, pp. 91-96, Jul. 1987.

I. Bentov and M. J. Reed, "The effect of aging on the cutaneous microvasculature," Microvasc. Res., vol. 100, pp. 25-31, 2015

Bhattacharyya, A. et al., 2018. A novel approach for automated detection of focal EEG signals using empirical wavelet transform. Neural Computing and Applications, 29(8), pp.47-57.

Bhoi, A.K., Sherpa, K.S. & Khandelwal, B., 2017. Arrhythmia and ischemia classification and clustering using QRS-ST-T (QT) analysis of electrocardiogram. Cluster Computing, 21(1), pp.1033-1044.

Bigarella, C.L., Liang, R. & Ghaffari, S., 2014. Stem cells and the impact of ROS signaling. Development (Cambridge), 141(22), pp.4206-4218.

T. S. Blacker and M. R. Duchen, "Investigating mitochondrial redox state using NADH and NADPH autofluorescence," Free Radic. Biol. Med., vol. 100, no. Supplement C, pp. 53-65, Nov. 2016.

Blackstone, E., Morrison, M. & Roth, M.B., 2005. H2S induces a suspended animation-like state in mice. Science, 308(5721), p.518.

Bleys, J., Navas-Acien, A. & Guallar, E., 2008. Serum selenium levels and all-cause, cancer, and cardiovascular mortality among US adults. Archives of Internal Medicine, 168(4), pp.404-410.

Brandes, R.P., Weissmann, N. & Schroder, K., 2014. Nox family NADPH oxidases in mechano-transduction: Mechanisms and consequences. Antioxidants and Redox Signaling, 20(6), pp.887-898.

M. Butawan, R. L. Benjamin, and R. J. Bloomer, "Methylsulfonylmethane: Applications and Safety of a Novel Dietary Supplement," Nutrients, vol. 9, no. 3, Mar. 2017.

Bos, E.M. et al., 2009. Hydrogen sulfide-induced hypometabolism prevents renal ischemia/reperfusion injury. Journal of the American Society of Nephrology, 20(9), pp.1901-1905.

Carlstrom, M., Wilcox, C.S. & Arendshorst, W.J., 2015. Renal autoregulation in health and disease. Physiological Reviews, 95(2), pp.405-511.

B. Chance, B. Schoener, R. Oshino, F. Itshak, and Y. Nakase, "Oxidation-reduction ratio studies of mitochondria in freeze-trapped samples. NADH and flavoprotein fluorescence signals," J. Biol. Chem., vol. 254, no. 11, pp. 4764-4771, Jun. 1979.

B. Chance, "Metabolic Heterogeneities in Rapidly Metabolizing Tissues," J. Appl. Cardiol., vol. 4, no. 4, pp. 207-221, 1989.

Chouchani E. T.,[1,2] Victoria R. Pell,[3] Andrew M. James,[4] Lorraine M. Work,[5] Kourosh Saeb-Parsy,[6] Christian Frezza,[7] Thomas Krieg,[3] and Michael P. Murphy[4] "A Unifying Mechanism for Mitochondrial Superoxide Production during Ischemia-Reperfusion Injury", Cell Metabolism 23, February 9, 2016.

Chouchani, E.T. et al., 2014. Ischaemic accumulation of succinate controls reperfusion injury through mitochondrial ROS. Nature, 515(7527), pp.431-435.

Chung H, Dai T, Sharma S, Huang Y, Carroll J, Hamblin M. The Nuts and Bolts of Low-level Laser (Light) Therapy. Ann Biomed Eng. 2012;40(2):516-533. doi:10.1007/s10439-011-0454-7.

C. E. Cooper and G. C. Brown, "The inhibition of mitochondrial cytochrome oxidase by the gases carbon monoxide, nitric oxide, hydrogen cyanide and hydrogen sulfide: chemical mechanism and physiological significance," J. Bioenerg. Biomembr., vol. 40, no. 5, pp. 533-539, Oct. 2008.

Dasgupta, A., 2014. Cardiac Markers. Clinical Chemistry, Immunology and Laboratory Quality Control, pp.127-144. https://doi.org/10.1016/B978-0-12-407821-5.00008-5.

Devasagayam TP, Tilak JC, Boloor KK, Sane KS, Ghaskadbi SS, Lele RD, "Free radicals and antioxidants in human health: current status and future prospects". The Journal of the Association of Physicians of India. 52: 794-804, 2004.

Di Gialleonardo V., David M. Wilson, and Kayvan R. Keshari, "The Potential of Metabolic Imaging", Semin. Nucl. Med., 46(1): 28-39, 2016.

Donald JA. Gasotransmitter Family. In: Handbook of Hormones. Elsevier; 2016:601. doi:doi.org/10.1016/B978-0-12-801028-0.00103-3.

Duan, J. et al.. Metabolic remodeling induced by mitokines in heart failure. Aging (Albany NY). 2019 Sep 15; 11(17): 7307-7327.

Ferraresi C., M. R. Hamblin, and N. A. Parizotto, "Low-level laser (light) therapy (LLLT) on muscle tissue: performance, fatigue and repair benefited by the power of light," Photonics Lasers Med., vol. 1, no. 4, pp. 267-286, Nov. 2012.

Ferrari R., MD, PhD; Cristina Balla, MD; Michele Malagù, MD; Gabriele Guardigli, MD; Giampaolo Morciano, MD, PhD; Matteo Bertini, MD; Simone Biscaglia, MD; Gianluca Campo, MD, "Reperfusion Damage - A Story of Success, Failure, and Hope", Circ J, 81: 131-141, 2017.

Fu M, Zhang W, Wu L, Yang G, Li H, Wang R., "Hydrogen sulfide (H2S) metabolism in mitochondria and its regulatory role in energy production", Proc Natl Acad Sci U S A., 109(8):2943-8, 2012.

Fuss T. L., B.A. and Leo L. Cheng, "Metabolic Imaging in Humans", Top Magn Reson Imaging, 25(5): 223-235, 2016.

R. A. Gatenby and R. J. Gillies, "Why do cancers have high aerobic glycolysis?," Nat. Rev. Cancer, vol. 4, no. 11, pp. 891-899, Nov. 2004.

Grimolizzi, F. & Arranz, L., 2018. Multiple faces of succinate beyond metabolism in blood. Haematologica, 103(10), pp.1586-1592.

Griendling, K.K. et al., 2016. Measurement of Reactive Oxygen Species, Reactive Nitrogen Species, and Redox-Dependent Signaling in the Cardiovascular System: A Scientific Statement from the American Heart Association, Circulation journal, 119,5,39-65.

Hamblin M., M. Victor Pires de Sousa and T. Agrawal, "Handbook of Low-Level Laser Therapy", Pan Stanford Publishing Pte. Ltd., Singapore, 2017.

Hamblin M., C. Ferraresi, Y.-Y. Huang, L. F. de Freitas, and J. Carroll, "Low-level light therapy: photobiomodulation", Editor: SPIE Press, Bellingham, Washington, USA, 2018.

Hayyan M, Hashim MA, AlNashef IM (March 2016). "Superoxide Ion: Generation and Chemical Implications". Chemical Reviews. 116 (5): 3029-85.

Hellmann, M., Roustit, M. & Cracowski, J.L., 2015. Skin microvascular endothelial function as a biomarker in cardiovascular diseases? Pharmacological Reports, 67(4), pp.803-810.

Huang, Ying-Ying, Carroll JD, H.M., 2009. Biphasic dose response. Dose response, pp.358-383. http://dx.doi.org/10.2203/dose-response.09-027.Hamblin

Hwang, J. et al., 2003. Pulsatile Versus Oscillatory Shear Stress Regulates NADPH Oxidase Subunit Expression: Implication for Native LDL Oxidation. Circulation Research, 93(12), pp.1225-1232.

Iwata, A. et al., 2015. Selenide Targets to Reperfusing Tissue and Protects It From Injury*. Read Online: Critical Care Medicine | Society of Critical Care Medicine, 43(7).

Jacques S., "How tissue optics affect dosimetry of photodynamic therapy", Journal of Biomedical Optics 15(5), 051608, 2010.

Jekell, A. et al., 2004. Elevated circulating levels of thioredoxin and stress in chronic heart failure. The european Journal of Heart failure, 6, pp.883-890.

Jackson, W.F., 2016. Arteriolar oxygen reactivity: where is the sensor and what is the mechanism of action? Journal of Physiology, 594(18), pp.5055-5077.

S. Kalinina et al., "Correlative NAD(P)H-FLIM and oxygen sensing-PLIM for metabolic mapping," J. Biophotonics, vol. 9, no. 8, pp. 800-811, Aug. 2016.

Kalogeris T., Christopher P. Baines1,2,3, Maike Krenz1,2, and Ronald J. Korthuis Ischemia/Reperfusion, Compr Physiol. ; 7(1): 113-170, 2017.

T. I. Karu, L. V. Pyatibrat, and N. I. Afanasyeva, "Cellular effects of low power laser therapy can be mediated by nitric oxide," Lasers Surg. Med., vol. 36, no. 4, pp. 307-314, Apr. 2005.

Kennedy R. T., et al., « Metabolic oscillations in □-cells », Diabetes, 51(S1), 2002.

Y. H. Kim, D. H. Kim, H. Lim, D.-Y. Baek, H.-K. Shin, and J.-K. Kim, "The anti-inflammatory effects of methylsulfonylmethane on lipopolysaccharide-induced inflammatory responses in murine macrophages," Biol. Pharm. Bull., vol. 32, no. 4, pp. 651-656, Apr. 2009.

Kindo, M. et al., 2016. Left Ventricular Transmural Gradient in Mitochondrial Respiration Is Associated with Increased Sub-Endocardium Nitric Oxide and Reactive Oxygen Species Productions. Frontiers in physiology, 7(August), pp.1-8.

Kindo, M. et al., 2012. Pressure overload-induced mild cardiac hypertrophy reduces left ventricular transmural differences in mitochondrial respiratory chain activity and increases oxidative stress. Frontier in physiology, 3(August), pp.1-14.

Kondo K, Bhushan S, King AL, Prabhu SD, Hamid T, Koenig S, et al. "H2S protects against pressure overload-induced heart failure via upregulation of endothelial nitric oxide synthase", Circulation, 127:1116-1127, 2013.

Kohn, C. et al., 2012. Hydrogen sulfide: Potent regulator of vascular tone and stimulator of angiogenesis. International Journal of Biomedical Science, 8(2), pp.81-86.

Kuganesan, M. et al., 2019. Selenium and hydrogen selenide: essential micronutrient and the fourth gasotransmitter? Intensive Care Medicine Experimental, 7(1). http://dx.doi.org/10.1186/s40635-019-0281-y.

Kumar, M., Pachori, R.B. & Acharya, U.R., 2017. Characterization of coronary artery disease using flexible analytic wavelet transform applied on ECG signals. Biomedical Signal Processing and Control, 31, pp.301-308.

Kvandal, P. et al., 2006. Low-frequency oscillations of the laser Doppler perfusion signal in human skin. Microvascular Research, 72(3), pp.120-127.

N. Lane, "Cell biology: power games," Nature, vol. 443, no. 7114, pp. 901-903, Oct. 2006.

Latham, F., 1951. THE OXYGEN PARADOX EXPERIMENTS ON THE EFFECTS OF OXYGEN IN HUMAN ANOXIA. The Lancet, 257(6646), pp.77-81

Li, S. & Yang, G., 2015. Hydrogen Sulfide Maintains Mitochondrial DNA Replication via Demethylation of TFAM. Antioxidants and Redox Signaling, 23(7), pp.630-642.

Li, Y.S.J., Haga, J.H. & Chien, S., 2005. Molecular basis of the effects of shear stress on vascular endothelial cells. Journal of Biomechanics, 38(10), pp.1949-1971.

Li, Y and Patrick J. Pagano, 2017. Microvascular NADPH oxidase in health and disease. Free Radic Biol Med., 109(1), pp.33-47.

Liebert, A. et al., 2017. A Role for Photobiomodulation in the Prevention of Myocardial Ischemic Reperfusion Injury: A Systematic Review and Potential Molecular Mechanisms. Scientific Reports, 7(February), pp.1-13. Available at:

http://dx.doi.org/10.1038/srep42386.

N. L. Lohr, A. Keszler, P. Pratt, M. Bienengraber, D. C. Warltier, and N. Hogg, "Enhancement of nitric oxide release from nitrosyl hemoglobin and nitrosyl myoglobin by red/near infrared radiation: potential role in cardioprotection," J. Mol. Cell. Cardiol., vol. 47, no. 2, pp. 256-263, Aug. 2009.

Loutzenhiser, R., Bidani, A. & Chilton, L., 2002. Renal myogenic response: Kinetic attributes and physiological role. Circulation Research, 90(12), pp.1316-1324.

Martelli F., Del Bianco S., Ismaelli A., "Light propagation through biological tissue and other diffusive media: theory, solutions, and software", Bellingham: Society of Photo-Optical Instrumentation Engineers, 2009.

Irma Martisiene, Regina Macianskiene, Voltage-Sensitive Fluorescence of Indocyanine Green in the Heart, Biophysical Journal, Volume 110, Issue 3, 2016, Pages 723-732, ISSN 0006-3495.

Moldogazieva, N.T. et al., 2018. ROS and RNS signalling: adaptive redox switches through oxidative/nitrosative protein modifications. Free Radical Research, 52(5), pp.507-543. Available at: https://doi.org/10.1080/10715762.2018.1457217.

Moss, N.G., Gentle, T.K. & Arendshorst, W.J., 2016. Modulation of the myogenic mechanism: Concordant effects of NO synthesis inhibition and O-2 dismutation on renal autoregulation in the time and frequency domains. American Journal of Physiology - Renal Physiology, 310(9), pp.F832-F845.

Nachiappan, Vasanthi; Muthukumar, Kannan, "Cadmium-induced oxidative stress in Saccharomyces cerevisiae". Indian Journal of Biochemistry and Biophysics. 47 (6), 2010.

Nagpure, B. V. & Bian, J.S., 2016. Interaction of Hydrogen Sulfide with Nitric Oxide in the Cardiovascular System. Oxidative Medicine and Cellular Longevity, 2016.

Nakamura, H., 2004. Thioredoxin as a Key Molecule in Redox Signaling. Antioxidants and Redox Signaling, 6(1), pp.15-17.

Nowak-Sliwinska P., J.-P. Ballini, G. Wagnières, and H. van den Bergh, "Processing of fluorescence angiograms for the quantification of vascular effects induced by anti-angiogenic agents in the CAM model," Microvasc. Res., vol. 79, no. 1, pp. 21-28, Jan. 2010.

P.T. Nowicki, S. Flavahan, H. Hassanain, S. Mitra, S. Holland, P.J. Goldschmidt-Clermont, N.A.F., 2001. Redox Signaling of the Arteriolar Myogenic Response. Circulation research, 89, pp. 114-116.

Olson, K.R., 2012. A practical look at the chemistry and biology of hydrogen sulfide. Antioxidants and Redox Signaling, 17(1), pp.32-44.

Papagiannakis A, Niebel B, Wit E, Heinemann M, Autonomous Metabolic Oscillations Robustly Gate the Early and Late Cell Cycle. Molecular Cell, Volume, 65, Issue 2, 19 January 2017, Pages 285-295, https://doi.org/10.1016/j.molcel.2016.11.018.

Park, H.C. et al., 2017. Effect of LED phototherapy on blood lactate level in Taekwondo contest. Mechanisms of Photobiomodulation Therapy XII, 10048(February 2017), p.1004801.

Patidar, S. & Pachori, R.B., 2014. Classification of cardiac sound signals using constrained tunable-Q wavelet transform. Expert Systems with Applications, 41(16), pp.7161-7170. Available at: http://dx.doi.org/10.1016/j.eswa.2014.05.052.

Patidar, S., Pachori, R.B. & Rajendra Acharya, U., 2015. Automated diagnosis of coronary artery disease using tunable-Q wavelet transform applied on heart rate signals. Knowledge-Based Systems, 82, pp.1-10. Available at: http://dx.doi.Org/10.1016/j.knosys.2015.02.011.

Pettersen, F.J. et al., 2016. Bioimpedance measurements of temporal changes in beating hearts. Biomedical Physics and Engineering Express, 2(6).

Petrenko, V. et al., 2020. In pancreatic islets from type 2 diabetes patients, the dampened circadian oscillators lead to reduced insulin and glucagon exocytosis. Proceedings of the National Academy of Sciences of the United States of America, 117(5), pp.2484-2495

Piper, H.M., 2000. The calcium paradox revisited: An artefact of great heuristic value. Cardiovascular Research, 45(1), pp.123-127

N. Ramanujam, R. Richards-Kortum, S. Thomsen, A. Mahadevan-Jansen, M. Follen, and B. Chance, "Low Temperature Fluorescence Imaging of Freeze-trapped Human Cervical Tissues," Opt. Express, vol. 8, no. 6, pp. 335-343, Mar. 2001.

V. Rapozzi, E. Della Pietra, S. Zorzet, M. Zacchigna, B. Bonavida, and L. E. Xodo, "Nitric oxide-mediated activity in anti-cancer photodynamic therapy," Nitric Oxide Biol. Chem., vol. 30, pp. 26-35, Apr. 2013.

Raddatz E, Thomas AC and al. Differential contribution of mitochondria, NADPH oxidases, and glycolysis to region-specific oxidant stress in the anoxic-reoxygenated embryonic heart. Am J Physiol Heart Circ Physiol 300: H820-H835, 2011.

K. J. Reeves, M. W. R. Reed, and N. J. Brown, "Is nitric oxide important in photodynamic therapy?," J. Photochem. Photobiol. B, vol. 95, no. 3, pp. 141-147, Jun. 2009.

Reglin, B. & Pries, A.R., 2014. Metabolic control of microvascular networks: Oxygen sensing and beyond. Journal of Vascular Research, 51(5), pp.376-392.

De Roever I, Bale G, Cooper RJ, Tachtsidis I." Functional NIRS Measurement of Cytochrome-C-Oxidase Demonstrates a More Brain-Specific Marker of Frontal Lobe Activation Compared to the Haemoglobins", Adv Exp Med Biol. 2017;977:141-147, 2017.

Sachar M. et al. "Protoporphyrin IX: the Good, the Bad, and the Ugly." The Journal of pharmacology and experimental therapeutics vol. 356,2 (2016): 267-75. doi:10.1124/jpet.115.228130.

Sedmera, D., Kucera, P. & Raddatz, E., 2002. Developmental changes in cardiac recovery from anoxia-reoxygenation. American Journal of Physiology - Regulatory Integrative and Comparative Physiology, 283(2 52-2), pp.379-388.

Shiogai, Y., Stefanovska, A. & McClintock, P.V.E., 2010. Nonlinear dynamics of cardiovascular ageing. Physics Reports, 488(2-3), pp.51-110.

Sies, H. & Jones, D.P., Reactive oxygen species (ROS) as pleiotropic physiological signalling agents. Nature Reviews Molecular Cell Biology. 2020 Available at: http://dx.doi.org/10.1038/s41580-020-0230-3.

M. C. Skala et al., "In vivo multiphoton microscopy of NADH and FAD redox states, fluorescence lifetimes, and cellular morphology in precancerous epithelia," Proc. Natl. Acad. Sci., vol. 104, no. 49, pp. 19494-19499, Dec. 2007.

M. Skala and N. Ramanujam, "Multiphoton Redox Ratio Imaging for Metabolic Monitoring in vivo," Methods Mol. Biol. Clifton NJ, vol. 594, pp. 155-162, 2010.

Smirnova, E. et al., 2013. Assessment of endothelial dysfunction in patients with impaired glucose tolerance during a cold pressor test. Diabetes and Vascular Disease Research, 10(6), pp.489-497.

Snijder P. M., A R Frenay, R A de Boer, A Pasch, J L Hillebrands, H G D Leuvenink and H van Goor, "Exogenous administration of thiosulfate, a donor of hydrogen sulfide, attenuates angiotensin II-induced hypertensive heart disease in rats", Br J Pharmacol. 2015 Mar; 172(6): 1494-1504.

Stefanovska, A., 2006. Coupled oscillators: Complex but not complicated cardiovascular and brain interactions. Annual International Conference of the IEEE Engineering in Medicine and Biology - Proceedings, pp.437-440

Szabo, C. et al., 2014. Regulation of mitochondrial bioenergetic function by hydrogen sulfide. Part I. Biochemical and physiological mechanisms. British Journal of Pharmacology, 171(8), pp.2099-2122.

Tamura, T., 2019. Current progress of photoplethysmography and SPO2 for health monitoring. Biomedical Engineering Letters, 9(1), pp.21-36. Available at: https://doi.org/10.1007/s13534-019-00097-w.

Tamura, T., 2018. Seamless Healthcare Monitoring, https://doi.org/10.1007/978-3-319-69362-0

Thomas DD. Breathing new life into nitric oxide signaling: A brief overview of the interplay between oxygen and nitric oxide. Redox Biol. 2015;5:225-233. doi:10.1016/j.redox.2015.05.002.

Ticcinelli, V. et al., 2017. Coherence and coupling functions reveal microvascular impairment in treated hypertension. Frontiers in Physiology, 8(OCT), pp.1-20.

Tretter, L., Patocs, A. & Chinopoulos, C., 2016. Succinate, an intermediate in metabolism, signal transduction, ROS, hypoxia, and tumorigenesis. Biochimica et Biophysica Acta - Bioenergetics, 1857(8), pp.1086-1101. Available at: http://dx.doi.org/10.1016/j.bbabio.2016.03.012.

Tsuruoka, N. et al., 2016. Lactate and glucose measurement in subepidermal tissue using minimally invasive microperfusion needle. Biomedical microdevices, (18:19), pp.2-9. http://dx.doi.org/10.1007/s10544-016-0049-z.

Tuchin V., "Tissue Optics and Photonics: Light-Tissue Interaction", J of Biomedical Photonics & Eng 1(2), pp 98-134, 2015.

Van Leeuwen, S.R., Baranoski, G.V.G. & Kimmel, B.W., 2017. Three-wavelength method for the optical differentiation of methemoglobin and sulfhemoglobin in oxygenated blood. Proceedings of the Annual International Conference of the IEEE Engineering in Medicine and Biology Society, EMBS, pp.4570-4573.

Arnoud H. M. van Vliet, Stefan Bereswill, and Johannes G. Kusters, "Ion Metabolism and Transport", in: Helicobacter pylori: Physiology and Genetics, Mobley HLT, Mendz GL, Hazell SL, editors, Washington (DC): ASM Press; 2001.

Wagnières G. et al., "In Vivo Fluorescence Spectroscopy and Imaging for Oncological Applications", Photochemistry and Photobiology, 68(5): 603-632, 1998.

A. Walsh, R. S. Cook, B. Rexer, C. L. Arteaga, and M. C. Skala, "Optical imaging of metabolism in HER2 overexpressing breast cancer cells," Biomed. Opt. Express, vol. 3, no. 1, pp. 75-85, Jan. 2012.

A. J. Walsh et al., "Optical metabolic imaging identifies glycolytic levels, subtypes, and early-treatment response in breast cancer," Cancer Res., vol. 73, no. 20, pp. 6164-6174, Oct. 2013.

G. Walther et al., "Metabolic syndrome individuals with and without type 2 diabetes mellitus present generalized vascular dysfunction: Cross-sectional study," Arterioscler. Thromb. Vasc. Biol., vol. 35, no. 4, pp. 1022-1029, 2015.

Wilson B., Patterson M., "The physics of photodynamic therapy", Phys. Med. Biol., (31)4, pp 327-360, 1986.

Wood K, Cortese-Krott M and al. Circulating Blood Endothelial Nitric Oxide Synthase Contributes to the Regulation of Systemic Blood Pressure and Nitrite Homeostasis. Arterioscler Thromb Vasc Biol. 2013;33:1861-1871.

Wu, M., Neilson, A., Swift, A.L., Moran, R., Tamagnine, J., Parslow, D., Armistead S., Lemire, K., Orrell, J., Teich, J., Chomicz, S., Ferrick, D.A., Multiparameter metabolic analysis reveals a close link between attenuated mitochondrial bioenergetic function and enhanced glycolysis dependency in human tumor cells. Am J Physiol Cell Physiol. 292, C125-C136, 2007.

Wu, D., Hu, Q. & Zhu, D., 2018. An update on hydrogen sulfide and nitric oxide interactions in the cardiovascular system. Oxidative Medicine and Cellular Longevity, 2018.

Yang, G., 2015. H2S epigenetic regulation of vascular cell functions. Cardiovascular Regenerative Medicine, pp.2-5.

Yu XH, Cui LB, Wu K, et al. Hydrogen sulfide as a potent cardiovascular protective agent. Clin Chim Acta. 2014;437:78-87. doi:10.1016/j.cca.2014.07.012.

Zhong, X. et al., 2018. Circadian Clock Regulation of Hepatic Lipid Metabolism by Modulation of m6A mRNA Methylation. Cell Reports, 25(7), p.1816-1828.e4.

Zhou, G. et al., 2016. Optimal ROS Signaling Is Critical for Nuclear Reprogramming. Cell Reports, 15(5), pp.919-925. Available at: http://dx.doi.org/10.1016/j.celrep.2016.03.084.

**Claims**

1. A device for applying Photobiomodulation (PBM) on a biological object comprising an EM source delivering light with an adequate temporal evolution of its optical power, said device also comprising a processing and/or a light control unit that determines the adequate temporal evolution of the optical power on the basis of the biological object optical coefficients and the light delivery geometry on/in the biological object, **characterized by** the fact that the PBM effects are induced by the generation of a specific fluence rate during a specific time, successively in each parts of the volume of the biological object.

2. A device according to claim 1 comprising a PBM monitoring and /or feedback system that is designed to adjust the optical power or irradiance based on the PBM effects.

3. A method for applying Photobiomodulation (PBM) on a biological object wherein light is delivered with an adequate temporal evolution of the optical power, the power being determined on the basis of the biological object optical coefficients and the light delivery geometry on/in the biological object, **characterized by** the fact that the PBM effects are furthermore induced by the generation of a specific fluence rate during a specific time, successively in each parts of the volume of the biological object.

4. Method according to claim 3 comprising an additional step consisting in the action of at least one exogenous stimulus.

5. Method according to claim 4 wherein the exogenous stimulus is an agent.

6. Method according to claim 5 wherein the exogenous agent is a $H_2S$ donor as STS compound.

7. Method according to claim 4 wherein the exogenous stimulus is a temperature change.

8. Method according to anyone of claims 3 to 7 comprising the action of at least two exogenous stimuli, one of them being an exogenous agent.

9. Method according to claim 5, 6 or 8 wherein light irradiation is starting at specific(s) moments during the exogenous agent(s) application.

10. Method according to anyone of claims 3 to 9 comprising a PBM monitoring and /or a feedback of the PBM effects, said method furthermore comprising the adjustment of the optical power, the delivery of light and/or the irradiance based on the PBM effects.

11. Method according to anyone of claims 3 to 10 wherein the optical power, the delivery of light and/or the irradiance is based on the measurement of one or several parameters reflecting the metabolic activity of the biological object.

12. Method according to anyone of claims 3 to 11 wherein the optical power, the delivery of light and/or the irradiance is based on the measurement, or on a frequency analysis of fluctuations, of one or several parameters reflecting the metabolic activity of the biological object.

13. Method according to anyone of claims 3 to 11 wherein the optical power, the delivery of light and/or the irradiance is based on a frequency analysis of fluctuations of one or several parameters reflecting the PBM effects on the biological object.

14. Method according to anyone of claims 3 to 13 wherein PBM is applied to trig changes in the metabolic activity of the biological object to enhance the effects of further PBMs.

15. Method according to anyone of claims 3 to 14 wherein the optical power, the delivery of light and/or the irradiance is used to adapt the amplitude, the phase and/or the frequency of fluctuations of one or several parameters reflecting the metabolic activity of the biological object.

16. Method according to anyone of claims 3 to 15 for the treatment of HCM cells comprising the application of a fluence rate of 3 mW/cm$^2$ during 3 minutes to all HCM cells.

17. Method for applying PBM on a biological object wherein light is delivered with an adequate temporal evolution of the optical power, **characterized by** the fact that the optical power, the delivery of light and/or the irradiance is based on the measurement, or on a frequency analysis of fluctuations, of one or several parameters reflecting the metabolic activity of the biological object.

18. Method for applying PBM on a biological object wherein light is delivered with an adequate temporal evolution of the optical power, **characterized by** the fact that the optical power, the delivery of light and/or the irradiance is based on a frequency analysis of fluctuations of one or several parameters reflecting the PBM effects on the biological object.

19. Use of the previous device or method as defined in any of the previous claims for the treatment of myocardial infarction (MI), including acute MI.

20. Use of the previous device or method as defined in any of the previous claims 1 to 15 for the treatment of biological objects subjected to ischemia and/or hypoxia / anoxia.

21. Use of the previous device or method as defined in any of the previous claims 1 to 18 for the treatment of acute respiratory distress syndrome (ARDS).

22. Use of the previous device or method as defined in any of the previous claims 1 to 18 for the treatment of desynchronized metabolic activities.

23. Use of the previous device or method as defined in any of the previous claims 1 to 18 for the treatment of desynchronized insulin secretion.

(Model and Monte-Carlo simulation: $\mu_a = 0.27$ mm$^{-1}$, $\mu_s = 18.7$ mm$^{-1}$, $g=0.81$, $k=4.87$, $\mu_{eff}^{-1} = 0.57$ mm) (S.L. JACQUES)

$$\frac{F(z)}{E} = e^{-\mu_{eff} z}$$

**Fig. 1**

**Fig. 2**

Fluorescence angiography image       Processed image

| Angiogenesis descriptors | Control no PBM | PBM | STS | PBM + STS | More developing angiogenesis corresponds to: |
|---|---|---|---|---|---|
| Branching points/mm$^2$ | 2400±200 | 3400±400 | 2700±200 | 4100±300 | a high value |
| The mean area of the vessel network meshes (10$^2$mm$^2$) | 6.7±1.5 | 2.9±0.6 | 3.8±0.4 | 4±0.5 | a low value |
| Mean of the 3$^{rd}$ quartile of mesh area histogram (10$^2$mm$^2$) | 5±1 | 3.1±0.5 | 1.8±0.4 | 2.1±0.3 | a low value |

Angiogenesis descriptors showing the effects of PBM performed at egg development day (EDD) 6 and observed at EDD 12. PBM was performed at 670nm with 15mW/cm$^2$ during 180 s. Note: the three descriptors are strongly correlated.

Fig. 3

Fig. 4

$$\mu_a = 0.27mm^{-1}, \mu_s = 18.7mm^{-1}, \mu_{\text{eff}} = 1.76mm^{-1}$$
$$g = 0.81, k = 4.87$$

Fig. 5

Fig. 6

Fig. 7

Fig. 8

EP 3 978 071 A1

Fig. 9

EP 3 978 071 A1

$$F' = 3mW.cm^{-2} \quad k = 4{,}87 \quad \mu_{eff} = 1{,}76\ mm^{-1}$$

$$E(z) = \frac{F'}{k} \times e^{(\mu_{eff} \times z)}$$

Fig. 10

$$F' = 3mW.cm^{-2} \Delta F' = 1{,}6mW.cm^{-2}\ k = 4{,}87 \quad \mu_{eff} = 1{,}76\ mm^{-1}$$

T = 180s

$$E(t) = \frac{F'}{k} \times e^{\left(\frac{\Delta F'}{F'} \times \frac{t}{T}\right)}$$

Fig. 11

**Fig. 12**

Apex
Left ventricule
iCAT
iCAT
Infarcted area

**Fig. 13**

Fig. 14

Fig. 15

Fig. 16

$$k = 4,87 \quad \mu_{eff} = 1,76 \ mm^{-1}$$

$F' = 15mW.cm^{-2}$
$\Delta_z = 0.15mm$

$F' = 3mW.cm^{-2}$
$\Delta_z = 0.3mm$

z (mm)

**Fig. 17**

$$k = 4,87 \quad \mu_{eff} = 1,76 \ mm^{-1}$$

$F' = 15mW.cm^{-2}$
$T = 40s$

$F' = 3mW.cm^{-2}$
$T = 180s$

t (s)

**Fig. 18**

Europäisches
Patentamt

European
Patent Office

Office européen
des brevets

# EUROPEAN SEARCH REPORT

**Application Number**

EP 20 19 9710

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/219604 A1 (YAROSLAVSKY ILYA [US] ET AL) 20 September 2007 (2007-09-20) * paragraphs [0013], [0054], [0089] - [0112], [0129] - [0131], [0156], [0176] - [0182]; figures 1,11,14 * | 1-23 | INV. A61N5/06 |
| X | US 2019/103571 A1 (DONG YAJIE [US] ET AL) 4 April 2019 (2019-04-04) * paragraphs [0003] - [0004], [0092]; claim 1; figure 8 * | 1,2 | |
| A | EP 3 668 276 A1 (SEABOROUGH LIFE SCIENCE B V [NL]) 17 June 2020 (2020-06-17) * claims 1-29 * | 1-23 | |

| | TECHNICAL FIELDS SEARCHED (IPC) |
|---|---|
| | A61N |

The present search report has been drawn up for all claims

| Place of search | Date of completion of the search | Examiner |
|---|---|---|
| Munich | 11 February 2021 | Gentil, Tamara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 19 9710

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2021

| Patent document cited in search report | | Publication date | Patent family member(s) | | Publication date |
|---|---|---|---|---|---|
| US 2007219604 | A1 | 20-09-2007 | US 2007219604 A1<br>US 2007219605 A1<br>WO 2007109245 A2 | | 20-09-2007<br>20-09-2007<br>27-09-2007 |
| US 2019103571 | A1 | 04-04-2019 | NONE | | |
| EP 3668276 | A1 | 17-06-2020 | EP 3668276 A1<br>WO 2020119965 A1 | | 17-06-2020<br>18-06-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **AALKJÆR, C. ; BOEDTKJER, D. ; MATCHKOV, V.** Vasomotion - what is currently thought?. *Acta Physiologica,* 2011, vol. 202 (3), 253-269
- **ACHARYA, U.R. et al.** Application of higher-order spectra for the characterization of Coronary artery disease using electrocardiogram signals. *Biomedical Signal Processing and Control,* 2017, vol. 31, 31-43, http://dx.doi.org/10.1016/j.bspc.2016.07.003
- **B. ALBERTS ; A. JOHNSON ; J. LEWIS ; M. RAFF ; K. ROBERTS ; P. WALTER.** Molecular Biology of the Cell. Garland Science, 2002
- **ÁLVAREZ, R.C. et al.** Cardiac Hemodynamic Monitoring in the Subcutaneous Space: A pre-clinical proof-of-concept. *MeMeA 2018 - 2018 IEEE International Symposium on Medical Measurements and Applications, Proceedings,* 2018
- **F. ANTUNES ; A. BOVERIS ; E. CADENAS.** On the mechanism and biology of cytochrome oxidase inhibition by nitric oxide. *Proc. Natl. Acad. Sci. U. S. A.,* November 2004, vol. 101 (48), 16774-16779
- **BAILEY, S.M. ; UDOH, U.S. ; YOUNG, M.E.** Circadian regulation of metabolism. *Journal of Endocrinology,* 2014, vol. 222 (2
- **K. A. BALL ; P. R. CASTELLO ; R. O. POYTON.** Low intensity light stimulates nitrite-dependent nitric oxide synthesis but not oxygen consumption by cytochrome c oxidase: Implications for phototherapy. *J. Photochem. Photobiol. B,* March 2011, vol. 102 (3), 182-191
- **S. BANERJEE ; D. K. BHATT.** Histochemical studies on the distribution of certain dehydrogenases in squamous cell carcinoma of cheek. *Indian J. Cancer,* March 1989, vol. 26 (1), 21-30
- **M. A. BEILKE ; C. COLLINS-LECH ; P. G. SOHNLE.** Effects of dimethyl sulfoxide on the oxidative function of human neutrophils. *J. Lab. Clin. Med.,* July 1987, vol. 110 (1), 91-96
- **I. BENTOV ; M. J. REED.** The effect of aging on the cutaneous microvasculature. *Microvasc. Res.,* 2015, vol. 100, 25-31
- **BHATTACHARYYA, A. et al.** A novel approach for automated detection of focal EEG signals using empirical wavelet transform. *Neural Computing and Applications,* 2018, vol. 29 (8), 47-57
- **BHOI, A.K. ; SHERPA, K.S. ; KHANDELWAL, B.** Arrhythmia and ischemia classification and clustering using QRS-ST-T (QT) analysis of electrocardiogram. *Cluster Computing,* 2017, vol. 21 (1), 1033-1044
- **BIGARELLA, C.L. ; LIANG, R. ; GHAFFARI, S.** Stem cells and the impact of ROS signaling. *Development (Cambridge),* 2014, vol. 141 (22), 4206-4218
- **T. S. BLACKER ; M. R. DUCHEN.** Investigating mitochondrial redox state using NADH and NADPH autofluorescence. *Free Radic. Biol. Med.,* November 2016, vol. 100 (C), 53-65
- **BLACKSTONE, E. ; MORRISON, M. ; ROTH, M.B.** H2S induces a suspended animation-like state in mice. *Science,* 2005, vol. 308 (5721), 518
- **BLEYS, J. ; NAVAS-ACIEN, A. ; GUALLAR, E.** Serum selenium levels and all-cause, cancer, and cardiovascular mortality among US adults. *Archives of Internal Medicine,* 2008, vol. 168 (4), 404-410
- **BRANDES, R.P ; WEISSMANN, N. ; SCHRODER, K.** Nox family NADPH oxidases in mechano-transduction: Mechanisms and consequences. *Antioxidants and Redox Signaling,* 2014, vol. 20 (6), 887-898
- **M. BUTAWAN ; R. L. BENJAMIN ; R. J. BLOOMER.** Methylsulfonylmethane: Applications and Safety of a Novel Dietary Supplement. *Nutrients,* March 2017, vol. 9 (3
- **BOS, E.M. et al.** Hydrogen sulfide-induced hypometabolism prevents renal ischemia/reperfusion injury. *Journal of the American Society of Nephrology,* 2009, vol. 20 (9), 1901-1905
- **CARLSTROM, M. ; WILCOX, C.S. ; AREND-SHORST, W.J.** Renal autoregulation in health and disease. *Physiological Reviews,* 2015, vol. 95 (2), 405-511
- **B. CHANCE ; B. SCHOENER ; R. OSHINO ; F. ITSHAK ; Y. NAKASE.** Oxidation-reduction ratio studies of mitochondria in freeze-trapped samples. NADH and flavoprotein fluorescence signals. *J. Biol. Chem.,* June 1979, vol. 254 (11), 4764-4771
- **B. CHANCE.** Metabolic Heterogeneities in Rapidly Metabolizing Tissues. *J. Appl. Cardiol.,* 1989, vol. 4 (4), 207-221
- **CHOUCHANI E. T.,1 ; 2 VICTORIA R. PELL ; 3 ANDREW M. JAMES ; 4 LORRAINE M. WORK ; 5 KOUROSH SAEB-PARSY,6 ; CHRISTIAN FREZZA,7 ; THOMAS KRIEG,3 ; MICHAEL P. MURPHY4.** A Unifying Mechanism for Mitochondrial Superoxide Production during Ischemia-Reperfusion Injury. *Cell Metabolism,* 09 February 2016, vol. 23

- **CHOUCHANI, E.T. et al.** Ischaemic accumulation of succinate controls reperfusion injury through mitochondrial ROS. *Nature,* 2014, vol. 515 (7527), 431-435
- **CHUNG H ; DAI T ; SHARMA S ; HUANG Y ; CARROLL J ; HAMBLIN M.** The Nuts and Bolts of Low-level Laser (Light) Therapy. *Ann Biomed Eng.,* 2012, vol. 40 (2), 516-533
- **C. E. COOPER ; G. C. BROWN.** The inhibition of mitochondrial cytochrome oxidase by the gases carbon monoxide, nitric oxide, hydrogen cyanide and hydrogen sulfide: chemical mechanism and physiological significance. *J. Bioenerg. Biomembr.,* October 2008, vol. 40 (5), 533-539
- **DASGUPTA, A.** Cardiac Markers. Clinical Chemistry. *Immunology and Laboratory Quality Control,* 2014, 127-144, https://doi.org/10.1016/B978-0-12-407821-5.00008-5
- **DEVASAGAYAM TP ; TILAK JC ; BOLOOR KK ; SANE KS ; GHASKADBI SS ; LELE RD.** Free radicals and antioxidants in human health: current status and future prospects. *The Journal of the Association of Physicians of India,* 2004, vol. 52, 794-804
- **DI GIALLEONARDO V. ; DAVID M. WILSON ; KAYVAN R. KESHARI.** The Potential of Metabolic Imaging. *Semin. Nucl. Med.,* 2016, vol. 46 (1), 28-39
- Gasotransmitter Family. **DONALD JA.** Handbook of Hormones. Elsevier, 2016, 601
- **DUAN, J. et al.** Metabolic remodeling induced by mitokines in heart failure. *Aging (Albany NY),* 15 September 2019, vol. 11 (17), 7307-7327
- **FERRARESI C. ; M. R. HAMBLIN ; N. A. PARIZOTTO.** Low-level laser (light) therapy (LLLT) on muscle tissue: performance, fatigue and repair benefited by the power of light. *Photonics Lasers Med.,* November 2012, vol. 1 (4), 267-286
- **FERRARI R., MD, PHD ; CRISTINA BALLA, MD ; MICHELE MALAGÙ, MD ; GABRIELE GUARDIGLI, MD ; GIAMPAOLO MORCIANO, MD, PHD ; MATTEO BERTINI, MD ; SIMONE BISCAGLIA, MD ; GIANLUCA CAMPO, MD.** Reperfusion Damage - A Story of Success, Failure, and Hope. *Circ J,* 2017, vol. 81, 131-141
- **FU M ; ZHANG W ; WU L ; YANG G ; LI H ; WANG R.** Hydrogen sulfide (H2S) metabolism in mitochondria and its regulatory role in energy production. *Proc Natl Acad Sci U S A.,* 2012, vol. 109 (8), 2943-8
- **FUSS T. L., B.A. ; LEO L. CHENG.** Metabolic Imaging in Humans. *Top Magn Reson Imaging,* 2016, vol. 25 (5), 223-235
- **R. A. GATENBY ; R. J. GILLIES.** Why do cancers have high aerobic glycolysis?. *Nat. Rev. Cancer,* November 2004, vol. 4 (11), 891-899
- **GRIMOLIZZI, F. ; ARRANZ, L.** Multiple faces of succinate beyond metabolism in blood. *Haematologica,* 2018, vol. 103 (10), 1586-1592
- **GRIENDLING, K.K. et al.** Measurement of Reactive Oxygen Species, Reactive Nitrogen Species, and Redox-Dependent Signaling in the Cardiovascular System: A Scientific Statement from the American Heart Association. *Circulation journal,* 2016, vol. 119 (5), 39-65
- **HAMBLIN M. ; M. VICTOR PIRES DE SOUSA ; T. AGRAWAL.** Handbook of Low-Level Laser Therapy. Pan Stanford Publishing Pte. Ltd, 2017
- **HAMBLIN M. ; C. FERRARESI ; Y.-Y. HUANG ; L. F. DE FREITAS ; J. CARROLL.** Low-level light therapy: photobiomodulation. 2018
- **HAYYAN M ; HASHIM MA ; AINASHEF IM.** Superoxide Ion: Generation and Chemical Implications. *Chemical Reviews,* March 2016, vol. 116 (5), 3029-85
- **HELLMANN, M. ; ROUSTIT, M. ; CRACOWSKI, J.L.** Skin microvascular endothelial function as a biomarker in cardiovascular diseases?. *Pharmacological Reports,* 2015, vol. 67 (4), 803-810
- **HUANG ; YING-YING ; CARROLL JD, H.M.** Biphasic dose response. *Dose response,* 2009, 358-383, http://dx.doi.org/10.2203/dose-response.09-027.Hamblin
- **HWANG, J. et al.** Pulsatile Versus Oscillatory Shear Stress Regulates NADPH Oxidase Subunit Expression: Implication for Native LDL Oxidation. *Circulation Research,* 2003, vol. 93 (12), 1225-1232
- Selenide Targets to Reperfusing Tissue and Protects It From Injury*. **IWATA, A. et al.** Read Online: Critical Care Medicine. Society of Critical Care Medicine, 2015, vol. 43
- **JACQUES S.** How tissue optics affect dosimetry of photodynamic therapy. *Journal of Biomedical Optics,* 2010, vol. 15 (5), 051608
- **JEKELL, A. et al.** Elevated circulating levels of thioredoxin and stress in chronic heart failure. *The european Journal of Heart failure,* 2004, vol. 6, 883-890
- **JACKSON, W.F.** Arteriolar oxygen reactivity: where is the sensor and what is the mechanism of action?. *Journal of Physiology,* 2016, vol. 594 (18), 5055-5077
- **S. KALININA et al.** Correlative NAD(P)H-FLIM and oxygen sensing-PLIM for metabolic mapping. *J. Biophotonics,* August 2016, vol. 9 (8), 800-811
- **KALOGERIS T. ; CHRISTOPHER P ; BAINES1,2,3 ; MAIKE KRENZ1,2 ; RONALD J.** Korthuis Ischemia/Reperfusion. *Compr Physiol.,* 2017, vol. 7 (1), 113-170
- **T. I. KARU ; L. V. PYATIBRAT ; N. I. AFANASYEVA.** Cellular effects of low power laser therapy can be mediated by nitric oxide. *Lasers Surg. Med.,* April 2005, vol. 36 (4), 307-314
- **KENNEDY R. T. et al.** Metabolic oscillations in □-cells. *Diabetes,* 2002, vol. 51 (S1

- **Y. H. KIM ; D. H. KIM ; H. LIM ; D.-Y. BAEK ; H.-K. SHIN ; J.-K. KIM.** The anti-inflammatory effects of methylsulfonylmethane on lipopolysaccharide-induced inflammatory responses in murine macrophages. *Biol. Pharm. Bull.,* April 2009, vol. 32 (4), 651-656
- **KINDO, M. et al.** Left Ventricular Transmural Gradient in Mitochondrial Respiration Is Associated with Increased Sub-Endocardium Nitric Oxide and Reactive Oxygen Species Productions. *Frontiers in physiology,* 07 August 2016, 1-8
- **KINDO, M. et al.** Pressure overload-induced mild cardiac hypertrophy reduces left ventricular transmural differences in mitochondrial respiratory chain activity and increases oxidative stress. *Frontier in physiology,* 03 August 2012, 1-14
- **KONDO K ; BHUSHAN S ; KING AL ; PRABHU SD ; HAMID T ; KOENIG S et al.** H2S protects against pressure overload-induced heart failure via upregulation of endothelial nitric oxide synthase. *Circulation,* 2013, vol. 127, 1116-1127
- **KOHN, C. et al.** Hydrogen sulfide: Potent regulator of vascular tone and stimulator of angiogenesis. *International Journal of Biomedical Science,* 2012, vol. 8 (2), 81-86
- **KUGANESAN, M. et al.** Selenium and hydrogen selenide: essential micronutrient and the fourth gasotransmitter?. *Intensive Care Medicine Experimental,* 2019, vol. 7 (1, http://dx.doi.org/10.1186/s40635-019-0281-y
- **KUMAR, M. ; PACHORI, R.B ; ACHARYA, U.R.** Characterization of coronary artery disease using flexible analytic wavelet transform applied on ECG signals. *Biomedical Signal Processing and Control,* 2017, vol. 31, 301-308
- **KVANDAL, P. et al.** Low-frequency oscillations of the laser Doppler perfusion signal in human skin. *Microvascular Research,* 2006, vol. 72 (3), 120-127
- **N. LANE.** Cell biology: power games. *Nature,* October 2006, vol. 443 (7114), 901-903
- **LATHAM, F.** THE OXYGEN PARADOX EXPERIMENTS ON THE EFFECTS OF OXYGEN IN HUMAN ANOXIA. *The Lancet,* 1951, vol. 257 (6646), 77-81
- **LI, S. ; YANG, G.** Hydrogen Sulfide Maintains Mitochondrial DNA Replication via Demethylation of TFAM. *Antioxidants and Redox Signaling,* 2015, vol. 23 (7), 630-642
- **LI, Y.S.J. ; HAGA, J.H. ; CHIEN, S.** Molecular basis of the effects of shear stress on vascular endothelial cells. *Journal of Biomechanics,* 2005, vol. 38 (10), 1949-1971
- **LI, Y ; PATRICK J. PAGANO.** Microvascular NADPH oxidase in health and disease. *Free Radic Biol Med.,* 2017, vol. 109 (1), 33-47
- **LIEBERT, A. et al.** A Role for Photobiomodulation in the Prevention of Myocardial Ischemic Reperfusion Injury: A Systematic Review and Potential Molecular Mechanisms. *Scientific Reports,* 07 February 2017, 1-13, http://dx.doi.org/10.1038/srep42386
- **N. L. LOHR ; A. KESZLER ; P. PRATT ; M. BIENENGRABER ; D. C. WARLTIER ; N. HOGG.** Enhancement of nitric oxide release from nitrosyl hemoglobin and nitrosyl myoglobin by red/near infrared radiation: potential role in cardioprotection. *J. Mol. Cell. Cardiol.,* August 2009, vol. 47 (2), 256-263
- **LOUTZENHISER, R. ; BIDANI, A. ; CHILTON, L.** Renal myogenic response: Kinetic attributes and physiological role. *Circulation Research,* 2002, vol. 90 (12), 1316-1324
- **MARTELLI F. ; DEL BIANCO S. ; ISMAELLI A.** Light propagation through biological tissue and other diffusive media: theory, solutions, and software. *Bellingham: Society of Photo-Optical Instrumentation Engineers,* 2009
- **IRMA MARTISIENE ; REGINA MAČIANSKIENĖ.** Voltage-Sensitive Fluorescence of Indocyanine Green in the Heart. *Biophysical Journal,* 2016, vol. 110 (3), ISSN 0006-3495, 723-732
- **MOLDOGAZIEVA, N.T. et al.** ROS and RNS signalling: adaptive redox switches through oxidative/nitrosative protein modifications. *Free Radical Research,* 2018, vol. 52 (5), 507-543, https://doi.org/10.1080/10715762.2018.1457217
- **MOSS, N.G. ; GENTLE, T.K. ; ARENDSHORST, W.J.** Modulation of the myogenic mechanism: Concordant effects of NO synthesis inhibition and O-2 dismutation on renal autoregulation in the time and frequency domains. *American Journal of Physiology - Renal Physiology,* 2016, vol. 310 (9), F832-F845
- **NACHIAPPAN ; VASANTHI ; MUTHUKUMAR ; KANNAN.** Cadmium-induced oxidative stress in Saccharomyces cerevisiae. *Indian Journal of Biochemistry and Biophysics,* 2010, vol. 47 (6
- **NAGPURE, B. V. ; BIAN, J.S.** Interaction of Hydrogen Sulfide with Nitric Oxide in the Cardiovascular System. *Oxidative Medicine and Cellular Longevity,* 2016
- **NAKAMURA, H.** Thioredoxin as a Key Molecule in Redox Signaling. *Antioxidants and Redox Signaling,* 2004, vol. 6 (1), 15-17
- **NOWAK-SLIWINSKA P. ; J.-P. BALLINI ; G. WAGNIÈRES ; H. VAN DEN BERGH.** Processing of fluorescence angiograms for the quantification of vascular effects induced by anti-angiogenic agents in the CAM model. *Microvasc. Res.,* January 2010, vol. 79 (1), 21-28

- **P.T. NOWICKI ; S. FLAVAHAN ; H. HASSANAIN ; S. MITRA ; S. HOLLAND ; P.J. GOLD-SCHMIDT-CLERMONT, N.A.F.** Redox Signaling of the Arteriolar Myogenic Response. *Circulation research,* 2001, vol. 89, 114-116
- **OLSON, K.R.** A practical look at the chemistry and biology of hydrogen sulfide. *Antioxidants and Redox Signaling,* 2012, vol. 17 (1), 32-44
- **PAPAGIANNAKIS A ; NIEBEL B ; WIT E ; HEINEMANN M.** Autonomous Metabolic Oscillations Robustly Gate the Early and Late Cell Cycle. *Molecular Cell,* 19 January 2017, vol. 65 (2), 285-295, https://doi.org/10.1016/j.molcel.2016.11.018
- **PARK, H.C. et al.** Effect of LED phototherapy on blood lactate level in Taekwondo contest. *Mechanisms of Photobiomodulation Therapy XII,* February 2017, vol. 10048, 1004801
- **PATIDAR, S. ; PACHORI, R.B.** Classification of cardiac sound signals using constrained tunable-Q wavelet transform. *Expert Systems with Applications,* 2014, vol. 41 (16), 7161-7170, http://dx.doi.org/10.1016/j.eswa.2014.05.052
- **PATIDAR, S. ; PACHORI, R.B. ; RAJENDRA ACHARYA, U.** Automated diagnosis of coronary artery disease using tunable-Q wavelet transform applied on heart rate signals. *Knowledge-Based Systems,* 2015, vol. 82, 1-10, http://dx.doi.Org/10.1016/j.knosys.2015.02.011
- **PETTERSEN, F.J. et al.** Bioimpedance measurements of temporal changes in beating hearts. *Biomedical Physics and Engineering Express,* 2016, vol. 2 (6
- **PETRENKO, V. et al.** In pancreatic islets from type 2 diabetes patients, the dampened circadian oscillators lead to reduced insulin and glucagon exocytosis. *Proceedings of the National Academy of Sciences of the United States of America,* 2020, vol. 117 (5), 2484-2495
- **PIPER, H.M.** The calcium paradox revisited: An artefact of great heuristic value. *Cardiovascular Research,* 2000, vol. 45 (1), 123-127
- **N. RAMANUJAM ; R. RICHARDS-KORTUM ; S. THOMSEN ; A. MAHADEVAN-JANSEN ; M. FOLLEN ; B. CHANCE.** Low Temperature Fluorescence Imaging of Freeze-trapped Human Cervical Tissues. *Opt. Express,* March 2001, vol. 8 (6), 335-343
- **V. RAPOZZI ; E. DELLA PIETRA ; S. ZORZET ; M. ZACCHIGNA ; B. BONAVIDA ; L. E. XODO.** Nitric oxide-mediated activity in anti-cancer photodynamic therapy. *Nitric Oxide Biol. Chem.,* April 2013, vol. 30, 26-35
- **RADDATZ E ; THOMAS AC.** Differential contribution of mitochondria, NADPH oxidases, and glycolysis to region-specific oxidant stress in the anoxic-reoxygenated embryonic heart. *Am J Physiol Heart Circ Physiol,* 2011, vol. 300, H820-H835
- **K. J. REEVES ; M. W. R. REED ; N. J. BROWN.** Is nitric oxide important in photodynamic therapy?. *J. Photochem. Photobiol. B,* June 2009, vol. 95 (3), 141-147
- **REGLIN, B. ; PRIES, A.R.** Metabolic control of microvascular networks: Oxygen sensing and beyond. *Journal of Vascular Research,* 2014, vol. 51 (5), 376-392
- **DE ROEVER I ; BALE G ; COOPER RJ ; TACHTSIDIS I.** Functional NIRS Measurement of Cytochrome-C-Oxidase Demonstrates a More Brain-Specific Marker of Frontal Lobe Activation Compared to the Haemoglobins. *Adv Exp Med Biol.,* 2017, vol. 977, 141-147
- **SACHAR M. et al.** Protoporphyrin IX: the Good, the Bad, and the Ugly. *The Journal of pharmacology and experimental therapeutics,* 2016, vol. 356 (2), 267-75
- **SEDMERA, D. ; KUCERA, P. ; RADDATZ, E.** Developmental changes in cardiac recovery from anoxia-reoxygenation. *American Journal of Physiology - Regulatory Integrative and Comparative Physiology,* 2002, vol. 283 (2 52-2), 379-388
- **SHIOGAI, Y. ; STEFANOVSKA, A. ; MCCLINTOCK, P.V.E.** Nonlinear dynamics of cardiovascular ageing. *Physics Reports,* 2010, vol. 488 (2-3), 51-110
- **SIES, H. ; JONES, D.P.** Reactive oxygen species (ROS) as pleiotropic physiological signalling agents. *Nature Reviews Molecular Cell Biology,* 2020, http://dx.doi.org/10.1038/s41580-020-0230-3
- **M. C. SKALA et al.** In vivo multiphoton microscopy of NADH and FAD redox states, fluorescence lifetimes, and cellular morphology in precancerous epithelia. *Proc. Natl. Acad. Sci.,* December 2007, vol. 104 (49), 19494-19499
- **M. SKALA ; N. RAMANUJAM.** Multiphoton Redox Ratio Imaging for Metabolic Monitoring in vivo. *Methods Mol. Biol. Clifton NJ,* 2010, vol. 594, 155-162
- **SMIRNOVA, E. et al.** Assessment of endothelial dysfunction in patients with impaired glucose tolerance during a cold pressor test. *Diabetes and Vascular Disease Research,* 2013, vol. 10 (6), 489-497
- **SNIJDER P. M. ; A R FRENAY ; R A DE BOER ; A PASCH ; J L HILLEBRANDS ; H G D LEUVENINK ; H VAN GOOR.** Exogenous administration of thiosulfate, a donor of hydrogen sulfide, attenuates angiotensin II-induced hypertensive heart disease in rats. *Br J Pharmacol.,* March 2015, vol. 172 (6), 1494-1504
- **STEFANOVSKA, A.** Coupled oscillators: Complex but not complicated cardiovascular and brain interactions. *Annual International Conference of the IEEE Engineering in Medicine and Biology - Proceedings,* 2006, 437-440

- **SZABO, C. et al.** Regulation of mitochondrial bioenergetic function by hydrogen sulfide. Part I. Biochemical and physiological mechanisms. *British Journal of Pharmacology,* 2014, vol. 171 (8), 2099-2122
- **TAMURA, T.** Current progress of photoplethysmography and SPO2 for health monitoring. *Biomedical Engineering Letters,* 2019, vol. 9 (1), 21-36, https://doi.org/10.1007/s13534-019-00097-w
- **TAMURA, T.** *Seamless Healthcare Monitoring,* 2018, https://doi.org/10.1007/978-3-319-69362-0
- **THOMAS DD.** Breathing new life into nitric oxide signaling: A brief overview of the interplay between oxygen and nitric oxide. *Redox Biol.,* 2015, vol. 5, 225-233
- **TICCINELLI, V. et al.** Coherence and coupling functions reveal microvascular impairment in treated hypertension. *Frontiers in Physiology,* 08 October 2017, 1-20
- **TRETTER, L. ; PATOCS, A. ; CHINOPOULOS, C.** Succinate, an intermediate in metabolism, signal transduction, ROS, hypoxia, and tumorigenesis. *Biochimica et Biophysica Acta - Bioenergetics,* 2016, vol. 1857 (8), 1086-1101, http://dx.doi.org/10.1016/j.bbabio.2016.03.012
- **TSURUOKA, N. et al.** Lactate and glucose measurement in subepidermal tissue using minimally invasive microperfusion needle. *Biomedical microdevices,* 2016, vol. 18:19, 2-9, http://dx.doi.org/10.1007/s10544-016-0049-z
- **TUCHIN V.** Tissue Optics and Photonics: Light-Tissue Interaction. *J of Biomedical Photonics & Eng,* 2015, vol. 1 (2), 98-134
- Three-wavelength method for the optical differentiation of methemoglobin and sulfhemoglobin in oxygenated blood. **VAN LEEUWEN, S.R. ; BARANOSKI, G.V.G. ; KIMMEL, B.W.** Proceedings of the Annual International Conference of the IEEE Engineering in Medicine and Biology Society. EMBS, 2017, 4570-4573
- Ion Metabolism and Transport. **ARNOUD H. M. VAN VLIET ; STEFAN BERESWILL ; JOHANNES G. KUSTERS.** Helicobacter pylori: Physiology and Genetics. ASM Press, 2001
- **WAGNIÈRES G. et al.** In Vivo Fluorescence Spectroscopy and Imaging for Oncological Applications. *Photochemistry and Photobiology,* 1998, vol. 68 (5), 603-632
- **A. WALSH ; R. S. COOK ; B. REXER ; C. L. ARTEAGA ; M. C. SKALA.** Optical imaging of metabolism in HER2 overexpressing breast cancer cells. *Biomed. Opt. Express,* January 2012, vol. 3 (1), 75-85
- **A. J. WALSH et al.** Optical metabolic imaging identifies glycolytic levels, subtypes, and early-treatment response in breast cancer. *Cancer Res.,* October 2013, vol. 73 (20), 6164-6174
- **G. WALTHER et al.** Metabolic syndrome individuals with and without type 2 diabetes mellitus present generalized vascular dysfunction: Cross-sectional study. *Arterioscler. Thromb. Vasc. Biol.,* 2015, vol. 35 (4), 1022-1029
- **WILSON B. ; PATTERSON M.** The physics of photodynamic therapy. *Phys. Med. Biol.,* 1986, vol. 31 (4), 327-360
- **WOOD K ; CORTESE-KROTT M.** Circulating Blood Endothelial Nitric Oxide Synthase Contributes to the Regulation of Systemic Blood Pressure and Nitrite Homeostasis. *Arterioscler Thromb Vasc Biol.,* 2013, vol. 33, 1861-1871
- **WU, M. ; NEILSON, A. ; SWIFT, A.L. ; MORAN, R. ; TAMAGNINE, J. ; PARSLOW, D. ; ARMISTEAD S. ; LEMIRE, K. ; ORRELL, J. ; TEICH, J.** Multiparameter metabolic analysis reveals a close link between attenuated mitochondrial bioenergetic function and enhanced glycolysis dependency in human tumor cells. *Am J Physiol Cell Physiol.,* 2007, vol. 292, C125-C136
- **WU, D. ; HU, Q. ; ZHU, D.** An update on hydrogen sulfide and nitric oxide interactions in the cardiovascular system. *Oxidative Medicine and Cellular Longevity,* 2018
- **YANG, G.** H2S epigenetic regulation of vascular cell functions. *Cardiovascular Regenerative Medicine,* 2015, 2-5
- **YU XH ; CUI LB ; WU K et al.** Hydrogen sulfide as a potent cardiovascular protective agent. *Clin Chim Acta.,* 2014, vol. 437, 78-87
- **ZHONG, X. et al.** Circadian Clock Regulation of Hepatic Lipid Metabolism by Modulation of m6A mRNA Methylation. *Cell Reports,* 2018, vol. 25 (7), 1816-1828
- **ZHOU, G. et al.** Optimal ROS Signaling Is Critical for Nuclear Reprogramming. *Cell Reports,* 2016, vol. 15 (5), 919-925, http://dx.doi.org/10.1016/j.celrep.2016.03.084